# EUROPÄISCHE PATENTSCHRIFT

(11) **EP 3 669 831 B1**
(45) Veröffentlichungstag und Bekanntmachung des Hinweises auf die Patenterteilung: **03.06.2026**
(21) Anmeldenummer: 18213421.3
(22) Anmeldetag: 18.12.2018
(51) Int. Cl.: A61F 5/01

(54) **MEDIZINISCHES HILFSMITTEL FÜR EIN GELENK EINER PERSON UND VERFAHREN ZUM BETRIEB EINES MEDIZINISCHEN HILFSMITTELS**
MEDICAL AID FOR A JOINT OF A PERSON AND METHOD FOR OPERATING A MEDICAL AID
APPAREIL MÉDICAL POUR UNE JOINTURE D'UNE PERSONNE ET PROCÉDÉ DE FONCTIONNEMENT D'UN APPAREIL MÉDICAL

(43) Veröffentlichungstag der Anmeldung: 24.06.2020
(73) Patentinhaber: medi GmbH & Co. KG, 95448 Bayreuth (DE)
(72) Erfinder: NAUMANN, Marco, 91257 Pegnitz (DE); LEHNER, Hans-Peter, 95463 Bindlach (DE)
(74) Vertreter: Lindner Blaumeier Patent- und Rechtsanwälte

(56) Entgegenhaltungen:
- WO-A1-2014/176353
- US-A- 5 429 140
- US-A1- 2002 146 672
- US-A1- 2010 116 277
- US-A1- 2016 213 924

## Beschreibung

Die Erfindung betrifft ein medizinisches Hilfsmittel für ein Gelenk einer Person, wobei das Hilfsmittel wenigstens zwei an dem Gelenk anzulegende, durch das Gelenk gegeneinander bewegliche Anteile und wenigstens eine Erfassungseinrichtung zur Erfassung einer Relativbewegung der Anteile aufweist, wie definiert im unabhängigen Patentanspruch 1.

Medizinische Hilfsmittel für unterschiedlichste Gelenke einer Person wurden im Stand der Technik bereits in vielfältiger Weise vorgeschlagen und umfassen beispielsweise Bandagen, Orthesen, Gurtsysteme, Strümpfe und sonstige, insbesondere kompressiv wirkende Kleidungsstücke. Dabei soll im Rahmen dieser Beschreibung "medizinisch" auch im Sinne von Sportanwendungen verstanden werden, die durch angestrebte positive Effekte für die Anatomie und/oder Gesundheit und/oder Leistungsfähigkeit, denen medizinische Überlegungen zugrunde liegen, gekennzeichnet sind. Medizinische Hilfsmittel dieser Art für Gelenke umfassen mithin beispielsweise auch Sportbandagen und dergleichen. Ferner soll im Rahmen der hiesigen Beschreibung auch die Wirbelsäule als Gelenk bzw. eine Gruppe von Gelenken aufgefasst werden, auf die medizinische Hilfsmittel angewendet werden können.

Das medizinische Hilfsmittel kann auch unabhängig von derartigen Behandlungseinrichtungen, die durch die eingangs genannten Anteile gebildet werden können oder diese umfassen können, existieren. Es kann beispielsweise explizit auf die Erfassung der Gelenkbewegung als Relativbewegung der Anteile ausgestaltet sein, mithin insbesondere eine Zusatzeinrichtung bzw. Zubehör für eine Behandlungseinrichtung wie eine Orthese, eine Bandage, einen Strumpf oder dergleichen bilden. Bei medizinischen Behandlungsmitteln oder auch konkret Behandlungseinrichtungen können die beiden Anteile durch getrennte Bauteile gebildet werden, die beispielsweise starr ausgebildet sind und gelenkig miteinander verbunden sind. Möglich ist es jedoch auch, dass die Anteile als Teilbereiche eines einzigen flexiblen Objekts, beispielsweise eines Strumpfs, gebildet sind.

Personen, die insbesondere auf eine therapeutische Behandlung und/oder den Schutz des Gelenks abzielende medizinische Hilfsmittel nutzen, tendieren dazu, Schonhaltungen bzw. Schonbewegungen einzunehmen bzw. durchzuführen, die zu nachteilhaften Effekten während der Behandlung mit dem medizinischen Hilfsmittel führen können. Umfasst das Hilfsmittel beispielsweise eine Orthese, die den Bewegungsbereich des Gelenks, beispielsweise eines Knies, einschränkt, wird üblicherweise nicht der volle durch die Orthese zugelassene Bewegungsbereich durch die Person genutzt, sondern ein deutlich geringerer Bewegungsbereich, der in seiner Ausdehnung auch den beispielsweise durch einen Therapeuten gewünschten Bewegungsbereich deutlich unterschreitet. Derartiges kann durch eine Erfassungseinrichtung des Hilfsmittels, die eine Relativbewegung der Anteile erfasst, festgestellt werden.

Beispielsweise offenbart WO 2016/176544 A1 eine Sensor- und Feedbackplattform zur Nutzung in orthotischen und prosthetischen Vorrichtungen. Dabei werden mittels Sensoren gemessene Parameter, die eine Bewegung beschreiben können, zur Auswertung und/oder zur Durchführung von Aktionen an Datenverarbeitungs- und/oder Datendarstellungseinheiten geliefert. Konkret wird dort vorgeschlagen, als Sensormechanismus einen induktiven Sensor zu verwenden, der mit leitfähigem Material in einem Band oder Streifen interagiert, um Sensordaten zu erzeugen, die den Ort des induktiven Sensors relativ zu dem Band/Streifen anzeigen.

Nachteilhafterweise kann über eine derartige Plattform jedoch nur im Nachhinein festgestellt werden, dass der genutzte Bewegungsbereich nicht einem gewünschten oder sogar maximal zulässigen Bewegungsbereich entsprochen hat, um dies danach der Person, die die Orthese oder Prothese nutzt, mitzuteilen. Hierdurch lassen sich nur bedingt Verbesserungen in der Ausnutzung des Bewegungsbereichs herbeiführen, da für die Person kein intuitiv verständlicher Zusammenhang zwischen der Auswertung und den zuvor vorgenommenen Bewegungen vorliegt. Die Nutzung von Schonhaltungen bzw. Schonbewegungen führt jedoch zu Defiziten in der Muskelstärke und/oder der Bewegungsfunktion beim Tragen eines medizinischen Hilfsmittels, was unerwünscht ist.

US 2002/0146672 A1 betrifft ein Verfahren und ein System zum individuellen Üben eines oder mehrerer Parameter der Handbewegung in einer Umgebung der virtuellen Realität, welches zudem leistungsbasierte Interaktion mit dem Benutzer zur Verbesserung der Nutzermotivation bereitstellt. Konkret kann ein Sensorhandschuh verwendet werden, um die Positionen von Patientenfingern und Handgelenksflexion zu messen. Gemessene Daten werden zur Auswertung weitergeleitet, insbesondere auch an ein Simulationsmodul der virtuellen Realität. So sollen beispielsweise virtuelle Simulationsübungen für Bewegungsbereiche durchführbar sein, um die Flexion und Extension der Finger zu verbessern. Es können Zielbereiche für die Übungen vorgegeben werden, wobei der neue Zielbereich immer gemäß der vorangehenden Leistung definiert werden kann.

WO 2014/176353 A1 betrifft eine tragbare Rehabilitations-Überwachungseinrichtung, die von Patienten und medizinischem Personal genutzt werden kann, um Fortschritte und Compliance in zu Hause durchgeführten Rehabilitationsprogrammen zu überwachen. Dabei wird eine Rehabilitations-Überwachungseinrichtung verwendet, die von dem zu überwachenden Subjekt während der Übungen getragen wird. Die Überwachungsvorrichtung umfasst dabei geeignete Sensoren, beispielsweise ein Goniometer, um den Bewegungsbereich zu überwachen. Die Überwachungsvorrichtung kann ferner einen Feedback-Anteil enthalten.

US 5 429 140 A betrifft ein Rehabilitationssystem, welches ein Kraft-Feedbackystem, beispielsweise einen Kraft-Feedback-Handschuh, nutzt, um virtuelle verformbare Objekte zu simulieren. Vor der Rehabilitation wird eine Messung an einem realen Objekt durchgeführt, so dass eine grundsätzliche Fähigkeit des Patienten festgestellt werden kann. Somit können Rehabilitationssteuersignale für die virtuellen Objekte erzeugt werden, so dass beispielsweise bei einem Handschuh der Nutzer das Gefühl erhält, wiederum ein echtes Objekt in der Hand zu halten. Es werden aufgenommene diagnostische Informationen gesammelt und Rehabilitationsinformationen werden genutzt, um Patientendaten zu aktualisieren.

US 2010/0116277 A1 betrifft ein schaltbares Gelenkbeschränkungssystem, bei dem Bewegungssensorsignale mit vorbestimmten Situationen verglichen werden und Gelenkbeschränkungen geschaltet werden, wenn bestimmte vordefinierte Situationen eintreten.

US 2016/0213924 A1 offenbart Patiententherapiesysteme und -verfahren, bei denen Stimulation verwendet wird. Ein Stimulationselektrodenpaar ist an eine Ware gekoppelt, die wiederum an einen Patienten gekoppelt werden kann, um drahtgebunden oder drahtlos Stimulation auszulösen. Ein Sensor ist mit einem Controller gekoppelt, wobei der Sensor auch Bewegungsbereichsdaten (ROM-Daten) erfassen kann, um aktives Feedback über den aktuellen Bewegungsbereich zu geben. Dadurch kann der Benutzer angeregt werden, Muskeln während einer Erholungsphase zu nutzen.

Der Erfindung liegt daher die Aufgabe zugrunde, eine verbesserte Möglichkeit anzugeben, Schonhaltungen zu vermeiden, insbesondere ohne dabei auf eine gegebenenfalls vorhandene Schutzfunktion einer Behandlungseinrichtung verzichten zu müssen.

Diese Aufgabe wird gemäß der Erfindung gelöst durch ein medizinisches Hilfsmittel gemäß Anspruch 1. Vorteilhafte Ausgestaltungen ergeben sich aus den Unteransprüchen.

Ein medizinisches Hilfsmittel der eingangs genannten Art weist erfindungsgemäß mithin eine Auswerteeinrichtung auf, welche umfasst:
- eine Ermittlungseinheit zur Ermittlung eines tatsächlich beim Tragen des Hilfsmittels genutzten Bewegungsbereichs des Gelenks aus Erfassungsdaten der Erfassungseinrichtung,
- eine Vergleichseinheit zum Vergleich des genutzten Bewegungsbereichs mit einem gewünschten Bewegungsbereich, und
- eine Unterstützungseinheit zum Ermitteln eines die Person zur möglichst weitgehenden Nutzung des gewünschten Bewegungsbereichs führenden Unterstützungshinweises in Abhängigkeit des Vergleichsergebnisses,

wobei das medizinische Hilfsmittel ferner eine Ausgabeeinrichtung zur Ausgabe des Unterstützungshinweises an die Person aufweist,
wobei die Auswerteeinrichtung zur Ansteuerung der Ausgabeeinrichtung zur Ausgabe des wenigstens einen Unterstützungshinweises während einer beurteilten Bewegung ausgebildet ist.

Hierbei ist die Unterstützungseinheit zur Auswahl
- wenigstens eines Ausgabeparameters wenigstens eines des wenigstens einen Unterstützungshinweises, wobei der Ausgabeparameter eine Intensität eines Tons und/oder eines Lichtsignals und/oder eine Lautstärke eines Tons ist,
- in Abhängigkeit eines aus dem Vergleichsergebnis ermittelten Nutzungsmaßes des gewünschten Bewegungsbereichs und/oder Annäherungsmaßes an eine Grenze des gewünschten Bewegungsbereichs ausgebildet.

Dabei gilt im Rahmen der vorliegenden Erfindung, dass auch, wie eingangs genannt, medizinisch orientierte Sport-Hilfsmittel als medizinische Hilfsmittel aufgefasst werden sollen. Das Hilfsmittel kann auch für die Wirbelsäule als Gruppe von Gelenken vorgesehen sein, so dass es sich mit anderen Worten um ein medizinisches Hilfsmittel für ein Gelenk oder eine Wirbelsäule handelt. Andere Gelenke, bei denen ein derartiges medizinisches Hilfsmittel einsetzbar ist, sind beispielsweise Kniegelenke und/oder Ellenbogengelenke. Im Bezug zu dem jeweiligen Gelenk ist auch jeweils der Bewegungsbereich zu parametrieren. Bei einem Einachs-Gelenk, wie beispielsweise einem Knie, kann der Bewegungsbereich durch Winkel definiert werden, zwischen denen die Bewegung stattfindet bzw. stattfinden soll. Beispielsweise kann also ein gewünschter Bewegungsbereich durch einen Zielwinkel in wenigstens einer Bewegungsrichtung, beispielsweise Flexion und/oder Extension bei einem Knie, definiert werden.

Das medizinische Hilfsmittel kann ein alleinstehendes Hilfsmittel sein, ist jedoch bevorzugt einer insbesondere durch die Anteile gebildeten bzw. diese umfassenden Behandlungseinrichtung wenigstens zugeordnet bzw. umfasst diese. Behandlungseinrichtungen können beispielsweise dann Bandagen, Orthesen, Kleidungsstücke, insbesondere Kompressionskleidungsstücke, Gurtsysteme und/oder Schienensysteme sein. Insbesondere ist es also denkbar, dass das Hilfsmittel sozusagen wenigstens einer Behandlungseinrichtung als Zusatzeinrichtung zugeordnet ist, worauf im Folgenden noch näher eingegangen wird.

Eine grundlegende Idee der vorliegenden Erfindung ist es also, die aktuelle Bewegung des Gelenks mittels der Erfassungseinrichtung nachzuverfolgen und, möglichst zeitaktuell, mit einem beispielsweise über eine Einstelleinrichtung vorgegebenen gewünschten Bewegungsbereich zu vergleichen, um hieraus bei Bedarf einen Unterstützungshinweis auszugeben, der die Person zur möglichst weitgehenden Nutzung des gewünschten Bewegungsbereichs führt. Mit anderen Worten erhält die Person durch das erfindungsgemäß vorgeschlagene medizinische Hilfsmittel nicht nur die Information, dass beispielsweise eine nicht gewollte Schonung vorliegt, sondern sie wird gleichzeitig zu einem gewünschten, insbesondere gezielt vorgebbaren Bewegungsbereich hingeführt, so dass eine Art Proaktivität gegeben ist.

Insbesondere kann also vorgesehen sein, falls Unterstützungshinweise nicht grundsätzlich immer ausgegeben werden sollen, dass in der Unterstützungseinheit wenigstens ein Hinweiskriterium vorgesehen ist, welches wenigstens das Vergleichsergebnis auswertet und bei dessen Erfüllung ein bzw. der Unterstützungshinweis ausgegeben wird. Dabei können unterschiedlichen Hinweiskriterien unterschiedliche Unterstützungshinweise zugeordnet sein und/oder es können unterschiedliche Unterstützungshinweise, insbesondere durch weitere Auswertung des Vergleichsergebnisses, bei Erfüllung eines bestimmten Hinweiskriteriums ermittelt werden.

Konkret kann das Hinweiskriterium wenigstens ein Nutzungsmaß mit einem Schwellwert vergleichen, beispielsweise also, inwieweit der gewünschte Bewegungsbereich ausgenutzt wird und/oder für welchen Anteil an Bewegungszyklen des Gelenks er hinreichend ausgenutzt wurde, wofür gegebenenfalls ein weiterer Schwellwert vorgesehen werden kann.

Mit anderen Worten kann also gesagt werden, dass die Unterstützungseinheit durch Auswertung des Vergleichsergebnisses dann, wenn das Nutzungsmaß zu gering ist, der Person eine Information derart zukommen lässt, dass diese zu einer besseren Ausnutzung der Bewegungsmöglichkeiten hin zum gewünschten Bewegungsbereich geleitet wird. Vorzugsweise kann hierbei eine die konkrete Art des Nutzungsmangels beschreibende Nutzungsmangelinformation aus dem Vergleichsergebnis ermittelt und bei der Parametrierung des Unterstützungshinweises, insbesondere möglicherweise auch des Ausgabezeitpunkts, berücksichtigt werden. Als Art des Nutzungsmangels kann beispielsweise bei einem Knie festgestellt werden, dass die Flexion zu gering ist, da ein zu großer Abstand vom Flexions-Zielwinkel, der den gewünschten Bewegungsbereich begrenzt, gegeben ist. Durch die Parametrierung des Unterstützungshinweises in Abhängigkeit einer Art des Nutzungsmangels wird der Person vorteilhaft vermittelt, wo Verbesserungspotential besteht. Eine bevorzugte Ausgestaltung sieht zudem vor, dass bei der Parametrierung des Unterstützungshinweises auch ein aktueller Bewegungszustand berücksichtigt wird, insbesondere zur zeitlichen Abstimmung des Unterstützungshinweises und/oder zur Anleitung der Person, aus dem Bewegungszustand zur besseren Ausnutzung des gewünschten Bewegungsbereichs zu finden.

Zusammenfassend wird allgemein der Unterstützungshinweis auf Basis des Vergleichsergebnisses so ausgestaltet und zeitlich angesetzt, dass er eine zu einer besseren Ausnutzung des gewünschten Bewegungsbereichs führende Information, insbesondere intuitiv und in der Bewegungssituation selbst, vermittelt.

Der gewünschte Bewegungsbereich, welcher bevorzugt mittels einer Einstelleinrichtung vorgebbar bzw. einstellbar ist, entspricht also letztlich einem Ziel, zu dem die Person, beispielsweise ein Patient oder Sportler, durch eine entsprechende Ausgestaltung der Unterstützungshinweise geleitet bzw. geführt wird. Insbesondere ist es somit möglich, einen Patienten aus einem Schonhaltungen bzw. Schonbewegungen entsprechenden, aktuell genutzten Bewegungsbereich zu einem therapeutisch sinnvollen, aktuell gewünschten Bewegungsbereich, der insbesondere vom Therapeuten individuell vorgegeben werden kann, hin zu führen.

Wie bereits dargelegt, kann das medizinische Hilfsmittel zwar einer Behandlungseinrichtung lediglich zugeordnet sein, weist jedoch bevorzugt die Behandlungseinrichtung auf, bildet diese also letztlich um eine zusätzliche Funktionalität weiter. Konkret kann vorgesehen sein, dass das Hilfsmittel, insbesondere als Behandlungseinrichtung und/oder die Anteile realisierend, wenigstens einen strumpfartigen, über das Gelenk zu ziehenden und/oder bandagenartig um das Gelenk zu wickelnden und/oder als Kleidungsstück und/oder als Gurtsystem ausgebildeten Umfassungsabschnitt und/oder wenigstens ein Gelenkführungselement, insbesondere wenigstens zwei gelenkig gekoppelte, starre Gelenkführungselemente, aufweist. Eine gegebenenfalls derart gebildete Behandlungseinrichtung kann mithin insbesondere eine Bandage, eine Orthese, ein Strumpf, eine Rückenschiene für die Wirbelsäule, ein Kleidungsstück und/oder ein Gurtsystem sein oder umfassen.

Dabei sei an dieser Stelle noch angemerkt, dass bei einer Zuordnung zu einer Behandlungseinrichtung diese nicht dauerhaft oder fest sein muss, sondern es durchaus denkbar ist, beispielsweise das medizinische Hilfsmittel, welches insbesondere lösbar mit einer Behandlungseinrichtung verbindbar ist, über eine gesamte Therapie oder dergleichen zu verwenden, beispielsweise zunächst mit einer Orthese als Behandlungseinrichtung, danach mit einer Bandage als Behandlungseinrichtung, darauf folgend mit einem Strumpf als Behandlungseinrichtung und schließlich mit einem Gurt oder Ähnlichem als Behandlungseinrichtung.

Konkret kann vorgesehen sein, dass die Erfassungseinrichtung wenigstens einen in wenigstens einen Anteil integrierten oder an wenigstens einem Anteil und/oder einem mit einem Anteil bewegten Körperbereich lösbar oder unlösbar befestigten oder befestigbaren Sensor umfasst. Dabei sei an dieser Stelle nochmals darauf hingewiesen, dass es sich bei den gegeneinander bewegbaren Anteilen nicht zwangsläufig um zwei getrennte Komponenten des Hilfsmittels bzw. einer Behandlungseinrichtung handeln muss, sondern es durchaus auch denkbar ist, eine flexible Komponente zu verwenden, wobei die Anteile dann durch Teilbereiche der Komponente gebildet werden. Derartige flexible Komponenten können beispielsweise den bereits erwähnten Strumpf, Bandagenelemente aus Kompressionsmaterial, flexible Schienen, Gurte und/oder dergleichen umfassen. Insbesondere dann, wenn die Anteile als Teil eines Umfassungsabschnitts realisiert sind, kann es zweckmäßig sein, Sensoren bzw. Sensoranteile der Erfassungseinrichtung bereits in die entsprechende Komponente, beispielsweise den Umfassungsabschnitt, zu integrieren bzw. gezielt Mittel zur bevorzugt wieder lösbaren Befestigung an der entsprechenden Komponente, konkret den jeweiligen Anteilen, vorzusehen. Bei der wieder lösbaren Befestigung können beispielsweise Klett-Befestigungsmittel herangezogen werden, aber auch Druckknöpfe und dergleichen. Dies ist insbesondere dann zweckmäßig, wenn durch die Anteile oder konkreter die Behandlungseinrichtung und die Art, wie sie angelegt werden bzw. wird, bereits geeignete Positionen für die Sensoren der Erfassungseinrichtung definiert werden, so dass letztlich über die Befestigungsmittel eine Befestigung an definierten Positionen an den entsprechenden Anteilen erfolgen kann.

In weiterer konkreter Ausgestaltung kann die Erfassungseinrichtung zur Messung eines Abstandes zwischen zwei Sensoren und/oder Sensoranteilen und/oder eines Gelenkwinkels und/oder wenigstens einer Dynamikgröße der Gelenkbewegung, insbesondere einer Winkelgeschwindigkeit und/oder einer Winkelbeschleunigung, ausgebildet sein. Handelt es sich beispielsweise um ein einachsiges Gelenk, erfolgt die Bewegung des Gelenks und somit der Anteile gegeneinander als eine Verschwenkung um diese Gelenkachse. Zwei Sensoren bzw. zwei Sensoranteile eines Sensors können den Abstand zwischen den Sensoren bzw. den Sensoranteilen vermessen und dadurch auf einen Gelenkwinkel bzw. im zeitlichen Verlauf auch Dynamikgrößen schließen. Selbstverständlich ist auch der Einsatz bei Gelenken mit mehreren Gelenkachsen denkbar, beispielsweise bei Kugelgelenken und dergleichen. Im Hinblick auf die Anwendung des medizinischen Hilfsmittels bei einer Wirbelsäule können auch komplexere Bewegungsräume definiert werden, insbesondere auch solche, wie die Bewegung mehrerer einzelner Wirbelgelenke gemeinsam abbilden.

Zweckmäßigerweise kann der wenigstens eine Sensor ein Magnetismus und/oder Induktion nutzendes Messprinzip aufweisen. Derartige Sensoren, wie beispielsweise in der eingangs genannten WO 2016/176544 A1 beschrieben, sind besonders geeignet, hinreichend genau die Bewegung der Anteile gegeneinander zu vermessen; selbstverständlich sind jedoch auch andere Sensorprinzipien denkbar, beispielsweise kapazitive Abstandsmesssensoren und dergleichen.

Es ist zweckmäßig, zumindest einige der Komponenten des medizinischen Hilfsmittels, insbesondere die Auswerteeinrichtung, entfernt von dem Gelenk, insbesondere also extern zu einer Behandlungseinrichtung, zu realisieren. Auch Teile der Erfassungseinrichtung können grundsätzlich entfernt vom Gelenk bzw. einer Behandlungseinrichtung vorgesehen sein. Während es zwar grundsätzlich denkbar ist, dann die Kommunikation zwischen derartigen räumlich getrennt realisierten Komponenten des medizinischen Hilfsmittels drahtgebunden zu realisieren, ist es im Rahmen der vorliegenden Erfindung bevorzugt, eine drahtlose Kommunikation zu verwenden, insbesondere eine Funkkommunikation, um die Person möglichst wenig zu behindern. Beispielsweise können proprietäre, aber auch standardisierte Kommunikationsverfahren zur drahtlosen Kommunikation eingesetzt werden, beispielsweise Bluetooth oder dergleichen. Eine derartige drahtlose Kommunikation kann beispielsweise zwischen der Auswerteeinrichtung und der Erfassungseinrichtung und/oder Unterkomponenten hiervon erfolgen.

Möglich ist es im Rahmen der vorliegenden Erfindung aber selbstverständlich auch, das gesamte medizinische Hilfsmittel, insbesondere also sowohl die Erfassungseinrichtung als auch die Auswerteeinrichtung und die Ausgabeeinrichtung, an dem Gelenk selbst anzuordnen, insbesondere an einer oder als Teil einer Behandlungseinrichtung. Dabei sind insbesondere auch kompakte Ausgestaltungen möglich, die dann bevorzugt haptische und/oder akustische Ausgabeeinrichtungen aufweisen.

Vorzugsweise kann das Hilfsmittel eine Einstelleinrichtung zum Einstellen des gewünschten Bewegungsbereichs des Gelenks, den die Person beim Tragen des Hilfsmittels nutzen soll, aufweisen. Während es im Rahmen der vorliegenden Erfindung grundsätzlich bevorzugt ist, eine manuelle Einstellung des gewünschten Bewegungsbereichs, beispielsweise durch einen Therapeuten oder einem sonstigen Benutzer, gegebenenfalls auch die Person selbst, zu erlauben, kann es in vielen Ausgestaltungen auch zweckmäßig sein, bevorzugt zusätzlich, oder aber auch alternativ, auch eine automatische Einstellung eines gewünschten Bewegungsbereichs durch die Einstelleinrichtung zu erlauben. Hierbei kann beispielsweise ein, gegebenenfalls auch durch die Erfassungseinrichtung selbst bestimmbarer, Therapiefortschritt und/oder ein vorgegebener Therapieplan herangezogen werden.

In Ausgestaltung der Erfindung kann die Einstelleinrichtung ein, insbesondere getrennt von einer durch die Anteile gebildeten Behandlungseinrichtung vorliegendes, elektronisches und/oder mechanisches Einstellmittel aufweisen, insbesondere ein handhaltbares Mobilgerät umfassen. Das bedeutet, die Einstelleinrichtung kann im Rahmen der Erfindung unterschiedlichst ausgestaltet werden. Insbesondere dann, wenn ein entsprechendes Einstellmittel der Einstelleinrichtung gelenknah, insbesondere an einer Behandlungseinrichtung, vorgesehen ist, kann der Einstellvorgang auch eine mechanische Komponente aufweisen, beispielsweise dadurch, dass das wenigstens eine Gelenk bzw. wenigstens die Einstelleinrichtung in eine den gewünschten Bewegungsbereich begrenzende Position gebracht wird, welche dann entsprechend abgespeichert werden kann. Besonders vorteilhaft ist es aber auch, insbesondere zur manuellen und/oder automatischen Einstellung des gewünschten Bewegungsbereichs, elektronische Einstellmittel vorzusehen, welche zumindest teilweise auch durch ein handhaltbares Mobilgerät umgesetzt sein können. Das handhaltbare Mobilgerät, beispielsweise ein Mobiltelefon, kann dabei beispielsweise eine Benutzerschnittstelle aufweisen, über welche gewünschte Bewegungsbereiche und/oder Kriterien zu deren automatischer Ermittlung eingegeben werden können. Hierfür kann bei einem Smartphone und/oder Tablet als handhaltbarem Mobilgerät beispielsweise ein entsprechendes Computerprogramm, also eine Applikation (App), vorgesehen werden.

Gerade im Hinblick auf eine derartige Ausgestaltung sieht eine besonders bevorzugte Weiterbildung der vorliegenden Erfindung vor, dass das Mobilgerät zusätzlich eine als wenigstens ein Teil der Auswerteeinrichtung ausgebildete Recheneinrichtung, auf der eine das Mobilgerät als Einstellmittel und als der Teil der Auswerteeinrichtung ausbildende Applikation vorliegt, aufweist. In einer beispielhaften Ausgestaltung kann also vorgesehen sein, dass das handhaltbare Mobilgerät, bevorzugt drahtlos, mit der Erfassungseinrichtung kommuniziert und die Erfassungsdaten der Erfassungseinrichtung mittels der Recheneinrichtung auswertet, insbesondere, um den Vergleich des genutzten Bewegungsbereichs mit einem gewünschten Bewegungsbereich vorzunehmen und zu ermitteln, ob ein Unterstützungshinweis ausgegeben werden soll und/oder wie der Unterstützungshinweis parametriert werden muss, um die Person zur möglichst weitgehenden Nutzung des gewünschten Bewegungsbereichs führen zu können. In diesem Kontext kann im Übrigen ein Ausgabemittel des handhaltbaren Mobilgeräts auch als Ausgabeeinrichtung dienen, beispielsweise also ein Lautsprecher zur akustischen Ausgabe eines Unterstützungshinweises und/oder ein insbesondere als Touchscreen ausgebildetes Display zur optischen Ausgabe eines Unterstützungshinweises genutzt werden. Auf diese Weise wird also die Ausstattung und/oder die Rechenleistung des handhaltbaren Mobilgeräts optimal ausgenutzt, um nur wenige Komponenten des medizinischen Hilfsmittels an dem Gelenk selbst vorzusehen. Beispielsweise ist es vorteilhaft, wenn lediglich die Erfassungseinrichtung sowie gegebenenfalls die Behandlungseinrichtung an dem Gelenk selbst anzuordnen sind.

Eine andere, gegebenenfalls zusätzlich zu einem handhaltbaren Mobilgerät als Einstellmittel verwendbare Ausgestaltung sieht vor, dass die Einstelleinrichtung, insbesondere umfassend wenigstens einen eine momentane Gelenkstellung als Begrenzung, beispielsweise Maximum oder Minimum, des gewünschten Bewegungsbereichs übernehmenden Einstellknopf, an der oder als Teil der Behandlungseinrichtung realisiert ist und/oder zur Auswertung von Erfassungsdaten der Erfassungseinrichtung zur Ermittlung einer momentanen Gelenkstellung als Begrenzung, beispielsweise Maximum oder Minimum, des gewünschten Bewegungsbereichs ausgebildet ist. In einer konkreten Weiterbildung ist es also denkbar, dass beispielsweise die Behandlungseinrichtung einen Einstellknopf, beispielsweise einen "Set"-Knopf, aufweist, um aktuelle Gelenkstellungen zur Definition des gewünschten Bewegungsbereichs heranzuziehen. Hierbei können insbesondere Erfassungsdaten der Erfassungseinrichtung genutzt werden, um die momentane Gelenkstellung identifizieren zu können, wobei selbstverständlich auch andere Ausgestaltungen denkbar sind. Es sei angemerkt, dass ein einem "Set"-Knopf (Einstellknopf) entsprechendes Bedienelement selbstverständlich auch auf einer Benutzeroberfläche eines handgehaltenen Mobilgeräts umgesetzt werden kann. In beiden Ausgestaltungen, also sowohl bei an der Behandlungseinrichtung bzw. an wenigstens einem Anteil vorgesehenem Einstellknopf und/oder bei über ein Benutzerinterface des Mobilgeräts realisiertem Einstellknopf, kann zusätzlich zu einem derartigen "Set"-Knopf auch ein "Reset"-Knopf vorgesehen werden, der beispielsweise bei einer erneuten Positionierung des medizinischen Hilfsmittels, konkret also wenigstens der Anteile, am Patienten genutzt werden kann, um alle Einstellungen zurückzusetzen und/oder beispielsweise Basisstellungen zur Kalibrierung der Erfassungseinrichtung anzuzeigen.

Dabei sei an dieser Stelle, insbesondere auch hinsichtlich einer manuellen Einstellung über die Einstelleinrichtung, angemerkt, dass selbstverständlich auch weitere Einstellungen über die Einstelleinrichtung bei entsprechender Ausbildung vorgenommen werden können, insbesondere die Einstellung von auf den Unterstützungshinweis bezogenen Unterstützungsparametern. Beispielsweise ist es über die beschriebenen Einstelleinrichtungen neben der Angabe eines gewünschten Bewegungsbereichs auch möglich, Anteile des gewünschten Bewegungsbereichs zu definieren, in denen eine Unterstützung tatsächlich erfolgen soll (vgl. auch das oben diskutierte Hinweiskriterium), zu welchen (auch relativen) Zeitpunkten eine Unterstützung erfolgen soll, was die allgemeine Intensität des Unterstützungshinweises sein soll, und dergleichen. Ferner kann vorgesehen sein, dass über die Einstelleinrichtung auch wenigstens ein weiterer, dem gewünschten Bewegungsbereich zugeordneter Bereichsparameter einstellbar ist, insbesondere ein den Zeitraum, über den der Vergleich ausgewertet werden soll, beschreibender Zeitparameter und/oder eine Bewegungsart, für die der gewünschte Bewegungsbereich Anwendung finden soll.

Wie bereits erwähnt, muss die Einstelleinrichtung nicht (ausschließlich) zur manuellen Einstellung des gewünschten Bewegungsbereichs geeignet sein, sondern kann auch eine zumindest teilweise automatische Ermittlung des gewünschten Bewegungsbereichs umsetzen. So sieht eine bevorzugte Weiterbildung der vorliegenden Erfindung vor, dass die Einstelleinrichtung zur zumindest teilweise automatischen Ermittlung des gewünschten Bewegungsbereichs durch Auswertung von personenspezifischen Vorgabedaten und/oder die Gelenkbewegung des Gelenks der Person in einem zurückliegenden Zeitabschnitt beschreibende, mit der Erfassungseinrichtung erfasste Historiendaten ausgebildet ist. Personenspezifische Vorgabedaten können beispielsweise den Gesundheitszustand und/oder den Therapiefortschritt und/oder eine gewünschte Art und/oder Geschwindigkeit der Behandlung beschreiben. Insbesondere können die Vorgabedaten auch einen Behandlungsplan enthalten, in dem unterschiedlichen Zeitabschnitten unterschiedliche gewünschte Bewegungsbereiche zugeordnet sind. Die Zeitabschnitte müssen dabei nicht absolut festgelegt sein, nachdem sich der Therapiefortschritt, beispielsweise durch Auswertung von Erfassungsdaten der Erfassungseinrichtung, auch nachverfolgen lässt. Beispielsweise kann dann, wenn die Gelenkbewegung des Gelenks der Person in einem zurückliegenden Zeitabschnitt beschreibende, mit der Erfassungseinrichtung als Erfassungsdaten erfasste Historiendaten anzeigen, dass der bislang vorgesehene gewünschte Bewegungsbereich hinreichend gut ausgenutzt wird, zu einem neuen Zeitabschnitt mit erweitertem gewünschten Bewegungsbereich fortgeschritten werden. Anders ausgedrückt kann also gesagt werden, dass der gewünschte Bewegungsbereich, der aktuell zu verwenden ist, abhängig vom Verhalten der Person und/oder von deren Therapiefortschritt abhängig automatisch angepasst werden kann.

In bevorzugten Ausgestaltungen der vorliegenden Erfindung kann die Unterstützungseinheit bei der Ermittlung auszugebender Unterstützungshinweise auch zur Verwendung des durch Erfassungsdaten der Erfassungseinrichtung beschriebenen aktuellen Bewegungszustandes und/oder der unmittelbar zurückliegenden Bewegungshistorie des Gelenks ausgebildet sein. Anders und teilweise konkreter ausgedrückt kann seitens der Unterstützungseinheit zur Ermittlung des Unterstützungshinweises und/oder eines Ausgabezeitpunkts des Unterstützungshinweises eine Bewegungsphase, insbesondere eine Annäherung des Bewegungszustands an eine Extremposition, und/oder eine Bewegungshistorie des Gelenks berücksichtigt werden. Bei den Unterstützungshinweisen, die zu einer besseren Ausnutzung des gewünschten Bewegungsbereichs führen sollen, ist der Zeitpunkt der Ausgabe bzw. der aktuelle Bewegungszustand bei der Ausgabe als relativ wichtig zu bewerten. Betrachtet man beispielsweise eine Kniebewegung, ist es zweckmäßig, die Person dann zur weiteren Streckung anzuregen, wenn sich das Bein ohnehin gerade im Streckprozess befindet. Entsprechend kann eine davon unterschiedliche, unterscheidbare Unterstützung im Fall der Beugung dann besonders zweckmäßig und einfach durch die Person zuordenbar sein, wenn der Unterstützungshinweis bei der Verlangsamung des Beugungsvorgangs hin zum entsprechenden Wendepunkt auftritt.

Daher ist es im Rahmen der vorliegenden Erfindung besonders zweckmäßig, anhand der Erfassungsdaten den aktuellen Bewegungszustand und/oder die unmittelbar zurückliegende Bewegungshistorie des Gelenks dahingehend auszuwerten, das Erreichen eines Wendepunkts und/oder Anhaltepunkts der Bewegung festzustellen bzw. zu prädizieren. Letztendlich kann allgemein gesagt werden, dass eine auf den aktuellen Bewegungszustand, insbesondere eine aktuelle Bewegungsphase, zugeschnittene und somit intuitiv durch die Person verständliche Unterstützung erfolgt. Bei einem Knie und/oder einem sonstigen Einachsgelenk als Gelenk kann hierzu insbesondere auch die Winkelgeschwindigkeit betrachtet werden, mithin der Unterstützungshinweis abhängig von der Winkelgeschwindigkeit ermittelt werden. So wird, wie bereits erläutert, kurz vor dem Umdrehen, also dem Wendepunkt, die Bewegung langsamer, was ein Anzeichen für das Nahen des Wendepunkts ist. Erst dann können beispielsweise Unterstützungshinweise zum Weiterschwenken gegeben werden. Andere Bewegungsphasen, beispielsweise beim Kniegelenk, können selbstverständlich auch im Rahmen der Unterstützung ermittelt und berücksichtigt werden, beispielsweise eine Schwungphase und/oder eine Abdrückphase.

Es kann vorgesehen sein, dass die Unterstützungseinheit zur Ermittlung des Unterstützungshinweises durch Beurteilung des Vergleichsergebnisses über einen vorgegebenen und/oder vorgebbaren, insbesondere durch einen mittels der Einstelleinrichtung eingegebenen Zeitparameter beschriebenen, Zeitraum, insbesondere definiert als eine Anzahl von Bewegungszyklen des Gelenks, ausgebildet ist. Möglich ist es im Rahmen der vorliegenden Erfindung mithin, einen gegebenenfalls benutzerseitig einstellbaren Zeitraum zu betrachten, um die Ausnutzung des gewünschten Bewegungsbereichs über diesen Zeitraum zu beurteilen und aufgrund dieser Beurteilung zu entscheiden, welche Art von Unterstützung die zweckmäßigste ist. Beispielsweise kann also eine Analyse über mehrere Zyklen erfolgen, um dann beispielsweise einen mittleren Nutzungsgrad des gewünschten Bewegungsbereichs und/oder wiederholt auftretende "Nutzungsfehler"/Nutzungsmängel festzustellen und so die Unterstützungshinweise auf eine fundierte Grundlage zu stellen. Insbesondere kann es dabei vorteilhaft sein, einen gleitenden Zeitraum zu betrachten, mithin die Beurteilungsergebnisse ständig aktuell zu halten. So ist eine robuste, fundierte Bestimmung der benötigten Unterstützung und eine entsprechende verlässliche Ausgestaltung von Unterstützungshinweisen möglich, ohne auf eine hinreichende Aktualität zu verzichten.

Im Rahmen der vorliegenden Erfindung ist vorgesehen, dass die Auswerteeinrichtung zur Ansteuerung der Ausgabeeinrichtung zur Ausgabe des wenigstens einen Unterstützungshinweises während einer beurteilten Bewegung ausgebildet ist. Zusätzlich ist auch eine Ausgabe nach der beurteilten Bewegung möglich. Die Ausgabe des Unterstützungshinweises nach einer beurteilten Bewegung erfolgt besonders vorteilhaft unmittelbar nach derselben. Die Beurteilung kann sich dabei auf den bereits erwähnten Zeitraum beziehen, wobei es dann besonders zweckmäßig ist, den angesprochenen "gleitenden Zeitraum" zu realisieren, um so ständig zeitaktuell auf zurückliegende Veränderungen, insbesondere Fortschritte, reagieren zu können. Während die Person also das Gelenk benutzt, erhält sie zumindest relativ unmittelbar Feedback zur Ausnutzung des gewünschten Bewegungsbereichs und bei der Bewegung insbesondere unmittelbar umsetzbare Informationen darüber, wie der gewünschte Bewegungsbereich besser ausgenutzt werden kann, in Form der Unterstützungshinweise. Im Beispiel der Kniebewegung kann beispielsweise beim Laufen zwar eine Beurteilung über mehrere, beispielsweise eins bis vier, Bewegungszyklen stattfinden, dann aber während der laufenden Bewegung das entsprechende Resultat, insbesondere zu geeigneten Zeitpunkten während eines Bewegungszyklus, ausgegeben werden. Wird beispielsweise in dem beurteilten Zeitraum als Beurteilungsergebnis festgestellt, dass bei der Extension der gewünschte Bewegungsbereich deutlich schlechter genutzt wird als bei der Flexion, können zeitaktuelle Unterstützungshinweise bei der Annäherung an die volle Streckung des Knies ausgegeben werden, die die Person zur weitergehenden Extension hinführen, während bei Annäherung an die maximale Flexion die Unterstützungshinweise von geringerer Intensität sein können, anzeigend, dass hier zwar noch Verbesserungsmöglichkeiten bestehen, aber die geringere Abweichung vorliegt, so dass der Person intuitiv vermittelt wird, dass zwar auch hinsichtlich der Flexion noch Verbesserungsmöglichkeiten bestehen, das maßgebliche Problem aber in der Extension liegt, auf die sich die Person dann beispielsweise zu durch den Unterstützungshinweis hervorgehobenen Zeitpunkten besonders konzentrieren kann.

Doch auch, wenn die Beurteilung nicht zwangsläufig über einen Zeitraum erfolgt, kann eine solche gezielte Unterstützung stattfinden. Beispielsweise kann während eines Flexionsvorgangs prädiziert werden, bis wohin die Flexion erfolgen wird, beispielsweise anhand einer eintretenden Reduzierung der Winkelgeschwindigkeit, um dann zu überprüfen, wie nah dieser prädizierte Winkel an der Grenze des gewünschten Bewegungsbereichs liegt, und so proaktiv Unterstützungshinweise auszugeben, die die Person zur weiteren Flexion führen, wenn ein, insbesondere relevanter, Unterschied zur Begrenzung des gewünschten Bewegungsbereichs gegeben ist. Eine derartige Ausgestaltung führt mithin zu einer bewegungsphasenindividuellen Unterstützung, die dedizierter, aber gegebenenfalls weniger robust bzw. stärkeren Schwankungen unterworfen ist als eine Beurteilung über einen Zeitraum mehrerer Bewegungszyklen des Gelenks.

Die Ausgabeeinrichtung kann ein haptisches und/oder akustisches und/oder optisches Ausgabemittel aufweisen. Gegebenenfalls sind jedoch auch andere Ausgabemöglichkeiten denkbar, beispielsweise ein olfaktorisches Ausgabemittel oder dergleichen. Bevorzugte Ausgestaltungen nutzen dabei die Ausgabe des Unterstützungshinweises als optisches und/oder akustisches, parametrierbares Ausgabesignal und/oder auch als haptisches Signal, worauf im Folgenden noch genauer eingegangen werden wird.

Die Erfindung sieht vor, dass die Unterstützungseinheit zur Auswahl
- wenigstens eines Ausgabeparameters wenigstens eines des wenigstens einen Unterstützungshinweises, wobei der Ausgabeparameter eine Intensität eines Tons und/oder eines Lichtsignals und/oder eine Lautstärke eines Tons ist,
- in Abhängigkeit eines aus dem Vergleichsergebnis ermittelten Nutzungsmaßes des gewünschten Bewegungsbereichs und/oder Annäherungsmaßes an eine Grenze des gewünschten Bewegungsbereichs ausgebildet ist. Es kann vorgesehen sein, dass die Unterstützungseinheit zur Auswahl wenigstens eines weiteren Ausgabeparameters, insbesondere der Frequenz der Wiederholung eines Tons und/oder eines Lichtsignals, des wenigstens eines des wenigstens einen Unterstützungshinweises in Abhängigkeit des Nutzungsmaßes und/oder Annäherungsmaßes ausgebildet ist. Der Unterstützungshinweis kann dabei insbesondere als ein pulsierendes bzw. grundsätzlich periodisches Signal ausgegeben werden, wobei beispielsweise die Periode und/oder die Intensität in Abhängigkeit des Vergleichsergebnisses angepasst werden können. Bei einem akustischen Signal kann beispielsweise ähnlich wie bei einem Parkwarner oder dergleichen vorgesehen werden, dass eine Wiederholfrequenz eines Tons bei stärkerer Annäherung an die Grenze des gewünschten Bewegungsbereichs erhöht wird und/oder das Erreichen mit einem durchgängigen Ton quittiert wird. Auch eine umgekehrte Ausgestaltung (Reduzierung der Wiederholfrequenz bei größerer Annäherung an die Grenze des gewünschten Bewegungsbereichs) ist im Rahmen der vorliegenden Erfindung denkbar, wenn dies für einen bestimmten Personenkreis intuitiver wirkt. Zusätzlich oder alternativ kann sich auch die Intensität, beispielsweise also die Lautstärke, erhöhen, je schlechter der gewünschte Bewegungsbereich ausgenutzt wird. Eine andere konkrete Ausgestaltung der vorliegenden Erfindung kann auch vorsehen, beispielsweise mehrere einzelne Lichtquellen in Form einer Skala als optische Ausgabemittel, beispielsweise nahe am Gelenk, insbesondere an wenigstens einem der Anteile und/oder einer Behandlungseinrichtung, vorzusehen. Je besser der gewünschte Bewegungsbereich ausgenutzt wird, desto mehr Lichtquellen der Skala können beispielsweise erhellt werden. In diesem Kontext ist im Übrigen auch ein zunehmendes Zuschalten bzw. Abschalten von durch Vibration den Unterstützungshinweis ausgebenden Ausgabemitteln am Gelenk denkbar.

Eine besonders vorteilhafte Ausgestaltung, die zusätzlich zu einer optischen und/oder akustischen Ausgabe des Unterstützungshinweises eingesetzt werden kann, sieht vor, dass das medizinische Hilfsmittel ferner, insbesondere als Teil der Ausgabeeinrichtung, wenigstens eine durch die Auswerteeinrichtung in Abhängigkeit des Vergleichsergebnisses ansteuerbare Stimulationseinrichtung für wenigstens einen dem Gelenk zugeordneten Muskel der Person und/oder wenigstens ein zur Veränderung der Relativposition der Anteile ausgebildetes, durch die Auswerteeinrichtung in Abhängigkeit des Vergleichsergebnisses ansteuerbares, elektrisches und/oder mechanisches Antriebsmittel aufweist. Eine derartige Stimulationseinrichtung kann beispielsweise ein Vibrationskissen, das insbesondere in einer Behandlungseinrichtung integriert sein kann, umfassen. Ein derartiges Vibrationskissen ist so positioniert, dass es relevante Muskelgruppen, deren Betätigung im aktuellen Bewegungszustand zu einer verbesserten Ausnutzung des gewünschten Bewegungsbereichs führt, gezielt stimuliert. Verlangsamt sich beispielsweise die Annäherung an eine Extremposition eines Bewegungszyklus deutlich zu früh, kann durch eine Stimulation von Muskeln durch die Stimulationseinrichtung eine Weiterführung der Bewegung gezielt erreicht bzw. angeregt werden, mithin ein äußerst zielgerichteter haptischer Unterstützungshinweis erzeugt werden.

Denkbar ist es jedoch auch, gezielt ein elektrisches und/oder mechanisches Antriebsmittel einzusetzen. Auf diese Weise kann ebenso ein deutlicher haptischer Unterstützungshinweis gegeben werden, um beispielsweise eine Bewegungsphase noch weiter fortzuführen als ursprünglich geplant, indem beispielsweise eine Winkelgeschwindigkeit durch Betrieb des Antriebsmittels und entsprechende Beeinflussung der Relativposition der Anteile leicht erhöht wird, insbesondere so in Grenzen gehalten, dass kein "Aufzwingen" einer hinreichend weiten Bewegung erfolgt, sondern zumindest hauptsächlich eine Hinweisfunktion erfüllt wird. Beispielsweise kann die Wirkung eines solchen haptischen Unterstützungshinweises auf maximal 1 bis 3° bei einer Schwenkbewegung reduziert werden. Möglich ist es im Übrigen auch, ein rein mechanisches Antriebsmittel zur Ausgabe eines haptischen Unterstützungshinweises zu verwenden. Hierbei kann beispielsweise die Freigabe einer eine bestimmte Bewegungsrichtung unterstützenden Feder in Betracht gezogen werden, das bedeutet, das Antriebsmittel kann eine eine Bewegungsrichtung mechanisch unterstützende Feder aufweisen. Die Unterstützungseinheit kann zur Ausgabe des Unterstützungshinweises die Feder beispielsweise freigeben und/oder arretieren.

Dabei sei an dieser Stelle angemerkt, dass die Unterstützungshinweise, welche ja während der Bewegung des Gelenks ausgegeben werden, in jedem Fall vorzugsweise so ausgestaltet sind, dass der Bewegungsablauf nicht unterbrochen und/oder gestört wird, sondern allenfalls unterstützt wird.

Eine besonders vorteilhafte Weiterbildung der vorliegenden Erfindung sieht vor, dass die Auswerteeinrichtung eine Klassifizierungseinheit aufweist, die anhand der Erfassungsdaten der Erfassungseinrichtung die aktuelle Bewegung in eine Bewegungsartklasse klassifiziert, wobei der Unterstützungshinweis nur bei Vorliegen einer der zugeordneten Bewegungsart entsprechenden Bewegungsartklasse ausgegeben wird. Beispielsweise können Historiendaten, insbesondere der letzten Bewegungszyklen, ausgewertet werden, um die Bewegungsart erkennen zu können, beispielsweise bezüglich eines Knies bzw. der Wirbelsäule als Gelenk bzw. Gelenke, ob gerade mit einem Kraftfahrzeug gefahren wird, Treppen gelaufen werden, eine ebene Strecke entlanggelaufen wird und dergleichen. Insbesondere kann der Unterstützungshinweis beispielsweise auf bestimmte Bewegungsartklassen beschränkt werden, möglich ist es aber selbstverständlich auch, zwischen Bewegungsartklassen derart zu differenzieren, dass beispielsweise unterschiedlichen Bewegungsartklassen unterschiedliche gewünschte Bewegungsbereiche und/oder eine unterschiedliche Art der Ermittlung der Unterstützungshinweise und/oder unterschiedliche Unterstützungshinweise zugeordnet sind. So kann beispielsweise vorgesehen sein, dass bei einer Anwahl wenigstens eines einer Bewegungsart zugeordneten gewünschten Bewegungsbereichs mittels der Einstelleinrichtung die Auswerteeinrichtung, insbesondere mittels der Klassifizierungseinheit, ausgebildet ist, den gewünschten Bewegungsbereich zum Vergleich nur bei Vorliegen einer der zugeordneten Bewegungsart entsprechenden Bewegungsartklasse heranzuziehen. Insbesondere ist es bei der Unterscheidung zwischen unterschiedlichen Bewegungsartklassen somit möglich, Unterstützungshinweise gezielt auf bestimmte Bewegungsarten anzupassen, um so insbesondere während einer Therapiephase optimal auf das Erreichen des Therapieergebnisses hinarbeiten zu können.

Eine Weiterbildung der vorliegenden Erfindung sieht ferner vor, dass das medizinische Hilfsmittel eine Begrenzungseinrichtung zur Begrenzung eines Bewegungsbereichs des Gelenks auf einen zulässigen Bewegungsbereich aufweist. Derartige Begrenzungseinrichtungen, die beispielsweise häufig bei Orthesen als medizinisches Hilfsmittel bzw. Behandlungseinrichtung vorgesehen sind, ermöglichen es also, nicht nur einen gewünschten Bewegungsbereich zu definieren, sondern auch einen maximal erlaubten Bewegungsbereich, mithin einen zulässigen Bewegungsbereich, festzulegen. In diesem Zusammenhang ist es besonders vorteilhaft, wenn die Einstelleinrichtung zur Übernahme des mittels der Begrenzungseinrichtung eingestellten zulässigen Bewegungsbereichs als gewünschter Bewegungsbereich ausgebildet ist. Mithin kann eine besonders einfache Festlegung des gewünschten Bewegungsbereichs als der zulässige Bewegungsbereich getroffen werden, wenn dieser maximal ausgenutzt werden soll.

Für das medizinische Hilfsmittel ist ein Verfahren zum Betrieb des medizinischen Hilfsmittels der erfindungsgemäßen Art für ein Gelenk einer Person, umfassend wenigstens zwei an dem Gelenk anzulegende, durch das Gelenk gegeneinander bewegliche Anteile und wenigstens eine Erfassungseinrichtung zur Erfassung einer Relativbewegung der Anteile, denkbar, wobei das Verfahren folgende Schritte aufweist:
- Ermittlung eines tatsächlich beim Tragen der Behandlungseinrichtung genutzten Bewegungsbereichs des Gelenks aus Erfassungsdaten der Erfassungseinrichtung,
- Vergleich des genutzten Bewegungsbereichs mit einem gewünschten Bewegungsbereich,
- Ermitteln eines die Person zur möglichst weitgehenden Nutzung des gewünschten Bewegungsbereichs führenden Unterstützungshinweises in Abhängigkeit des Vergleichsergebnisses, und
- Ausgabe des Unterstützungshinweises an die Person während einer beurteilten Bewegung.

Hierbei wählt die Unterstützungseinheit
- wenigstens einen Ausgabeparameter wenigstens eines des wenigstens einen Unterstützungshinweises, wobei der Ausgabeparameter eine Intensität eines Tons und/oder eines Lichtsignals und/oder eine Lautstärke eines Tons ist,
- in Abhängigkeit eines aus dem Vergleichsergebnis ermittelten Nutzungsmaßes des gewünschten Bewegungsbereichs und/oder Annäherungsmaßes an eine Grenze des gewünschten Bewegungsbereichs aus.

Sämtliche Ausführungen bezüglich des erfindungsgemäßen medizinischen Hilfsmittels lassen sich analog auf das denkbare Verfahren übertragen, so dass auch mit diesem die bereits genannten Vorteile erhalten werden können.

Insbesondere kann auch gemäß dem Verfahren vorgesehen sein, dass der gewünschte Bewegungsbereich, den die Person beim Tragen des Hilfsmittels nutzen soll, insbesondere aufgrund einer Benutzereingabe, an einer Einstelleinrichtung eingestellt wird.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine Skizze zur Erläuterung des der Erfindung zugrunde liegenden Problems und Lösungsansatzes,
- Fig. 2: eine schematische Prinzipskizze von Komponenten eines erfindungsgemäßen medizinischen Hilfsmittels,
- Fig. 3: eine Orthese als Ausführungsbeispiel eines erfindungsgemäßen medizinischen Hilfsmittels,
- Fig. 4: die Anwendung eines erfindungsgemäßen medizinischen Hilfsmittels bei einer Bandage,
- Fig. 5: ein handhaltbares Mobilgerät,
- Fig. 6: die Anwendung der vorliegenden Erfindung bei einer Rückenorthese,
- Fig. 7: einen Ablaufplan eines ersten Ausführungsbeispiels eines Verfahrens zum Betrieb eines medizinischen Hilfsmittels,
- Fig. 8: einen Ablaufplan eines zweiten Ausführungsbeispiels eines Verfahrens zum Betrieb eines medizinischen Hilfsmittels, und
- Fig. 9: eine Abhängigkeit einer Wiederholfrequenz eines Signals vom Nutzungsgrad.

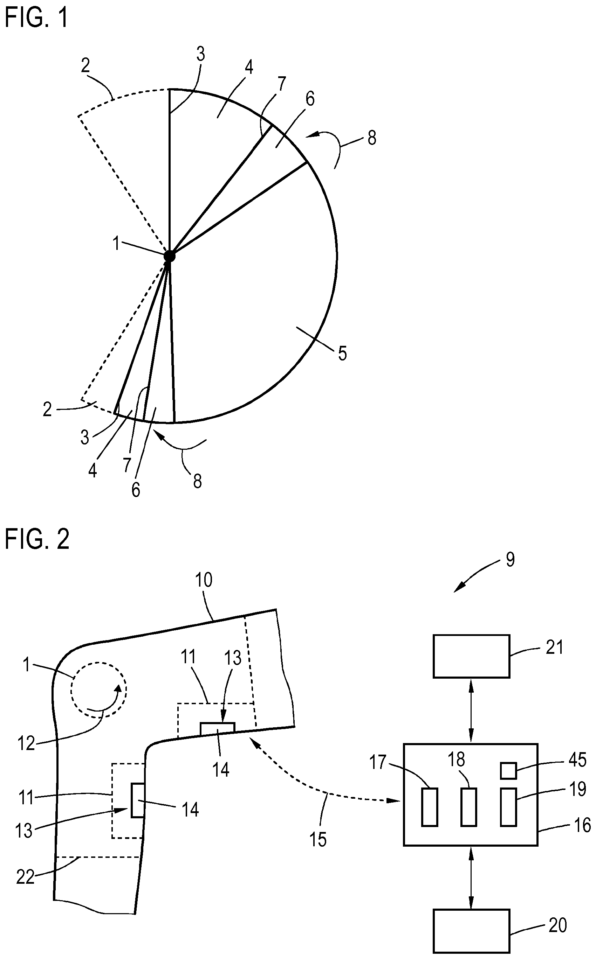

Fig. 1 erläutert die zugrunde liegende Idee der vorliegenden Erfindung anhand verschiedener Bewegungsbereiche (ROM - Ranges of Motion) eines durch den zentralen Punkt angedeuteten Gelenks 1 einer Person, beispielsweise eines Kniegelenks. Der gestrichelt angedeutete maximale Bewegungsbereich 2 des Gelenks 1 wird vorliegend durch eine Begrenzungseinrichtung einer verwendeten Behandlungseinrichtung, insbesondere einer Orthese, begrenzt, so dass sich ein durch die Linien 3 begrenzter zulässiger Bewegungsbereich 4 ergibt. Aufgrund von Schonhaltungen und Schonbewegungen während der Therapiephase nutzen Patienten bzw. allgemein Personen, die die genannte Behandlungseinrichtung einsetzen, nur einen kleinen Anteil des zulässigen Bewegungsbereichs 4, hier als der tatsächlich beim Tragen der Behandlungseinrichtung genutzte Bewegungsbereich 5 dargestellt.

Ferner existiert jedoch auch ein therapeutisch sinnvoll zu nutzender, gewünschter Bewegungsbereich 6, der durch die Begrenzungen 7 beschrieben werden kann. Die grundlegende Idee der vorliegenden Erfindung ist es nun, ein medizinisches Hilfsmittel, das der genannten Behandlungseinrichtung zugeordnet sein kann oder diese umfassen kann, so auszugestalten, dass der Patient anhand von durch Pfeile 8 symbolisierten Unterstützungshinweisen gezielt ausgehend von dem aktuell tatsächlich genutzten Bewegungsbereich 5 zur Nutzung größerer Anteile des gewünschten Bewegungsbereichs 6 geführt wird.

Fig. 2 zeigt eine schematische Prinzipskizze eines solchen erfindungsgemäßen medizinischen Hilfsmittels 9 für ein Gelenk 1, hier beispielsweise ein Kniegelenk oder sonstiges Einachsgelenk, einer nur angedeuteten Person 10. Ersichtlich sind am Körper der Person 10, dem Gelenk 1 benachbart, Anteile 11 vorgesehen, die durch die mittels des Pfeils angedeutete Bewegung des Gelenks 1 gegeneinander bewegt werden. Eine hier nur angedeutete Erfassungseinrichtung 13, die beispielsweise zwei Sensoren 14 bzw. Anteile eines Sensors 14 umfassen kann, die ausgebildet sind, die Relativposition und/oder Relativorientierung zwischen sich zu messen, vermisst die tatsächlich stattfindende Bewegung des Gelenks 1. Ihre Erfassungsdaten können über eine hier nur angedeutete Kommunikationsverbindung 15 an eine Auswerteeinrichtung 16 weitergeleitet werden. Die Auswerteeinrichtung 16 umfasst nun zunächst eine Ermittlungseinheit 17, die aus den Erfassungsdaten der Erfassungseinrichtung 13 den tatsächlich beim Tragen des medizinischen Hilfsmittels 9 genutzten Bewegungsbereich 5 des Gelenks 1 ermittelt, bei einem Einachsgelenk, wie hier beispielsweise dargestellt, insbesondere, indem der Gelenkwinkel nachvollzogen wird und die maximalen Winkel festgestellt werden. Es sei bereits an dieser Stelle angemerkt, dass die Erfassungseinrichtung 13 in ihren Erfassungsdaten auch Dynamikparameter beschreibt, im vorliegenden Beispiel eine Winkelgeschwindigkeit.

Eine Vergleichseinheit 18 der Auswerteeinrichtung 16 vergleicht den tatsächlich genutzten Bewegungsbereich 5 mit dem gewünschten Bewegungsbereich 6. Beispielsweise können die entsprechenden, den genutzten Bewegungsbereich 5 beschreibenden, insbesondere maximalen Gelenkwinkel mit den die Begrenzungen 7 beschreibenden maximalen und minimalen Gelenkwinkeln verglichen werden. Es ergibt sich letztlich wenigstens ein Nutzungsmaß, das anzeigt, inwieweit, insbesondere bezogen auf jede einzelne Begrenzung 7, der gewünschte Bewegungsbereich 6 auch verwendet wird.

Schließlich weist die Auswerteeinrichtung 16 auch eine Unterstützungseinheit 19 auf, welche einen Unterstützungshinweis in Abhängigkeit des Vergleichsergebnisses ermittelt. Der Unterstützungshinweis wird dabei so bestimmt, dass er die Person 10 zur möglichst weitgehenden Nutzung des gewünschten Bewegungsbereichs 6 führt bzw. leitet, was im Folgenden hinsichtlich konkreter Ausführungsbeispiele noch näher erläutert werden wird.

Dabei muss nicht zu jedem Zeitpunkt bzw. in jeder Situation ein Unterstützungshinweis ausgegeben werden, nachdem innerhalb der Unterstützungseinheit wenigstens ein Hinweiskriterium vorgesehen sein kann, welches wenigstens das Vergleichsergebnis auswertet und bei dessen Erfüllung ein bzw. der Unterstützungshinweis ausgegeben wird, ggf. nach geeigneter Parametrierung. Ferner kann allgemein gesagt werden, dass es im Hinblick auf die Führung der Person 10 zur weitgehenderen Nutzung des gewünschten Bewegungsbereichs 6 zweckmäßig ist, den aktuellen Bewegungszustand und zumindest die zurückliegende Bewegungshistorie ebenso bei der Ausgestaltung des Unterstützungshinweises zu berücksichtigen, insbesondere bei dessen konkreter Parametrierung, so dass im Unterstützungshinweis eine für die Person 10 intuitiv verständliche Anweisung enthalten ist, wie eine weitgehendere Nutzung des gewünschten Bewegungsbereichs 6 erzielt werden kann.

Der Unterstützungshinweis, der von der Unterstützungseinheit 19 ermittelt wurde, kann über eine entsprechende Ausgabeeinrichtung 20 ausgegeben werden. Diese kann zur optischen und/oder akustischen und/oder haptischen Ausgabe des Unterstützungshinweises beispielsweise entsprechende Ausgabemittel aufweisen, worauf im konkreten Beispiel noch näher eingegangen werden wird.

In bevorzugten Ausführungsbeispielen weist das erfindungsgemäße medizinische Hilfsmittel 9 ferner auch eine Eingabeeinrichtung 21 mit wenigstens einem Eingabemittel auf, über das der gewünschte Bewegungsbereich 6 manuell und/oder automatisch festgelegt werden kann, wobei das Zugrundelegen einer Benutzereingabe bevorzugt ist. Insbesondere kann ein Eingabemittel auch in mechanischer Form an den Anteilen 11 bzw. einer Behandlungseinrichtung 22, die in Fig. 2 ebenso angedeutet ist und auch zum medizinischen Hilfsmittel 9 gehören kann, als mechanisches Eingabemittel vorgesehen sein, beispielsweise im Fall der bezüglich Fig. 1 bereits angesprochenen Begrenzungseinrichtung, über welche auch mechanisch ein gewünschter Bewegungsbereich 6 festgelegt werden kann. Im Hinblick auf eine automatische Festlegung kann beispielsweise ein dynamischer oder festgelegter Therapieplan von einem Therapeuten als Benutzer für die Person 10 vorgegeben werden, der beispielsweise für verschiedene Therapiefortschritte den gewünschten Bewegungsbereich 6 in aufeinanderfolgenden Zeiträumen erweitert. Der Therapiefortschritt kann dabei einer festgelegten zeitlichen Abfolge folgen, bevorzugt ist es jedoch, über die Erfassungsdaten den Therapiefortschritt nachzuverfolgen, nachdem insbesondere das erwähnte Nutzungsmaß, welches die Vergleichseinheit 18 ermittelt, einen hervorragenden Anhaltspunkt liefert, da es angibt, inwieweit ein gewünschter Bewegungsbereich 6 bereits genutzt wird, so dass dies gegebenenfalls in Betracht gezogen werden kann, um zu einer nächsten Therapiestufe und somit einem erweiterten gewünschten Bewegungsbereich 6 fortschreiten zu können.

Ein derartiger Therapieplan kann im Übrigen auch die Nutzung unterschiedlicher Behandlungseinrichtungen 22 mit sich bringen, nachdem beispielsweise zunächst eine Orthese, dann eine Bandage und schließlich ein Gurtsystem oder dergleichen eingesetzt werden kann. Nachdem die Anteile und/oder der wenigstens eine Sensor bevorzugt lösbar an der Behandlungseinrichtung 22, zu der die Anteile 11 im Übrigen auch gehören können, befestigbar ist, beispielsweise über Klett-Befestigungsmittel, was auch für andere Komponenten des medizinischen Hilfsmittels 9 gelten kann, kann das medizinische Hilfsmittel 9 in bestimmten Ausgestaltungen mithin auch mit unterschiedlichen Behandlungseinrichtungen 22 genutzt werden.

Allgemein sei an dieser Stelle noch angemerkt, dass die Anteile 11 zur Behandlungseinrichtung 22 gehören können, aber nicht müssen. Die Anteile 11 können beispielsweise bei einer Orthese als Behandlungseinrichtung 22 starre Gelenkführungselemente sein, die gelenkig gekoppelt sind, möglich ist es aber auch, dass die Anteile 11 durch Teilbereiche eines flexiblen Bestandteils der Behandlungseinrichtung 22 bzw. des medizinischen Hilfsmittels 9 gebildet sind, bei einer Bandage beispielsweise aus Kompressionsmaterial.

Ferner gilt zunächst noch allgemein, dass die Auswerteeinrichtung 16, die Ausgabeeinrichtung 20 und die Einstelleinrichtung 21 wenigstens teilweise an der Behandlungseinrichtung 22, also gelenknah, realisiert sein können, und/oder auch wenigstens teilweise extern zur Behandlungseinrichtung 22, insbesondere also entfernt vom Gelenk 1, wobei dann die Kommunikationsverbindung 15 bevorzugt eine drahtlose Kommunikationsverbindung ist. Konkrete Ausgestaltungen werden im Folgenden noch näher erläutert werden.

Der Sensor 14 misst, wie beschrieben wurde, bevorzugt die relative Position und Orientierung zu dem anderen Sensor 14 bzw. der insbesondere passiven Sensorkomponente am anderen Anteil 11. Dabei können besonders bevorzugt magnetische und/oder induktive Messprinzipien eingesetzt werden, beispielsweise das Vorsehen von magnetischem Material an einem Anteil und eines entsprechenden Magnetfeldsensors am anderen Anteil 11. Andere denkbare Ausgestaltungen umfassen das Vorsehen von leitfähigem Material an einem Anteil 11 und von Induktionssensoren am anderen Anteil 11.

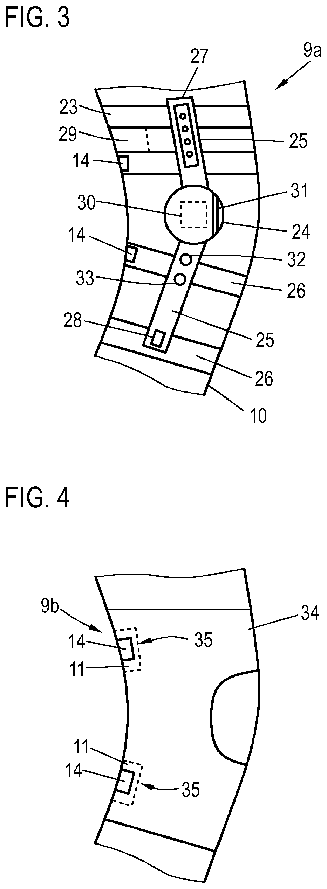

Fig. 3 zeigt ein erstes, konkretes Ausführungsbeispiel eines eine Orthese 23 als Behandlungseinrichtung 22 umfassenden erfindungsgemäßen medizinischen Hilfsmittels 9a, wobei vorliegend nur die am Gelenk 1, hier wiederum ein Kniegelenk, vorgesehenen Komponenten des medizinischen Hilfsmittels 9a gezeigt sind. Die Orthese weist vorliegend zwei starre, über ein Gelenk 24 gekoppelte Gelenkführungselemente 25 auf, die über Gurte 26 am Oberschenkel bzw. Unterschenkel der Person 10 gehaltert werden können. Die Sensoren 14 bzw. Sensorkomponenten des Sensors 14 sind vorliegend an den bezüglich des Kniegelenks inneren Gurten 26 angeordnet, können jedoch auch an den Gelenkführungselementen 25 vorgesehen sein. Die Sensorelemente 14, die die Erfassungseinrichtung 13 bilden, können beispielsweise in die Gurte 26 integriert sein.

Fig. 3 zeigt auch verschiedene mögliche Ausgestaltungen von Ausgabemitteln der Ausgabeeinrichtung 20. Zum einen gezeigt ist ein weniger bevorzugt optische Ausgabeelemente, bevorzugt haptische Ausgabeelemente aufweisendes skalaartiges Ausgabemittel 27, dessen Ausgabeelemente mit steigender Annäherung an eine vollständige Nutzung des gewünschten Bewegungsbereichs 6 beispielsweise nacheinander zugeschaltet werden können, um dies der Person 10 anzuzeigen. Als weiter bevorzugtes, akustisches Ausgabemittel weist die Ausgabeeinrichtung 20 gemäß Fig. 3 auch einen an der Orthese 23, hier einem der starren Gelenkführungselemente 25, angeordneten Lautsprecher 28 auf, über den Töne, gegebenenfalls auch Sprachausgaben, als Unterstützungshinweise ausgegeben werden können. Bevorzugt ist dabei eine Ausgestaltung mit wiederholten Tönen, deren Wiederholfrequenz und/oder Lautstärke und/oder Tonhöhe veränderbar ist, je nachdem, wie gut der gewünschte Bewegungsbereich 6 durch die Person 10 tatsächlich verwendet wird.

Als besonders intuitiv verständliche, denkbare Ausgestaltung hat sich dabei eine an einem Parkwarner eines Kraftfahrzeugs orientierte Ausbildung erwiesen, worin beispielsweise eine höhere Wiederholfrequenz eine stärkere Annäherung an die Begrenzungen 7 des gewünschten Bewegungsbereichs 6 anzeigt. Über die Tonhöhe kann beispielsweise die Begrenzung 7, beispielsweise also Richtung Flexion bzw. Richtung Extension, der Person 10 vermittelt werden. Dabei ist ohnehin zweckmäßig vorgesehen, die Ausgabe von einer aktuellen Bewegungsphase bzw. einem aktuellen Bewegungszustand abhängig zu machen, beispielsweise auf die Flexion bezogene Unterstützungshinweise dann auszugeben, wenn man sich dem Wendepunkt der Bewegung bei Extension nähert, und entsprechend für die Flexion.

Weitere bevorzugte, optionale haptische Ausgabemittel zeigt Fig. 3 in Form eines vorliegend am Oberschenkel vorgesehenen Vibrationskissens 29, über das relevante Muskelgruppen stimuliert werden können, beispielsweise als haptischer Hinweis, eine Bewegungsrichtung länger beizubehalten, und eines vorliegend elektrischen Antriebsmittels 30 an der Gelenkverbindung 24, welches in einem gewissen Rahmen, bevorzugt nur hinweisartig, die Bewegung in einer Richtung, beispielsweise bei Verlangsamung derselben, noch etwas forcieren kann, um einen Hinweis an die Person 10 auszugeben, dass hier noch weiterer gewünschter Bewegungsspielraum besteht.

Fig. 3 zeigt ferner angedeutet eine Begrenzungseinrichtung 31 an der Gelenkverbindung 24, über welche ein zulässiger Bewegungsbereich 4 eingestellt werden kann. Wie bereits erläutert wurde, kann die Begrenzungseinrichtung 31 auch als mechanisches Einstellmittel der Einstelleinrichtung 21 verstanden werden, welches vorliegend über einen Einstellknopf 32, beispielsweise einen SET-Knopf, ergänzt werden kann. Beispielsweise kann zunächst ein gewünschter Bewegungsbereich 6 über die Begrenzungseinrichtung 31 eingestellt werden, indem der SET-Knopf 32 genutzt wird, woraufhin es immer noch möglich ist, den zulässigen Bewegungsbereich 4 gegenüber dem gewünschten Bewegungsbereich 6 durch nachfolgende Verstellung der Begrenzungseinrichtung 31 noch zu erweitern. Ein ferner vorgesehener RESET-Knopf 33 kann ebenso vorhanden sein. Er kann beispielsweise bei einem Neuanlagen der Orthese 23 eingesetzt werden.

Fig. 4 zeigt den Einsatz eines medizinischen Hilfsmittels 9b bei einer Bandage 34 als Behandlungseinrichtung 22, wobei die Bandage 34 hier nicht zwangsläufig zu dem medizinischen Hilfsmittel 9b gehören muss. Gezeigt sind von diesem vorliegend der Übersichtlichkeit halber lediglich der wenigstens eine Sensor 14 sowie sensorseitige Komponenten eines Befestigungsmittels 35, hier eines Klettmittels, die die Anteile 11 bilden. Mittels der Befestigungsmittel 35 sind der wenigstens eine Sensor 14 sowie die Anteile 11 mithin lösbar an der Bandage 34 an insbesondere vorgegebenen Positionen befestigbar, wobei die weiteren Komponenten des Hilfsmittel 9b (Auswerteeinrichtung 16, Ausgabeeinrichtung 20 und Eingabeeinrichtung 21) als extern zum Gelenk vorgesehen nicht gezeigt sind.

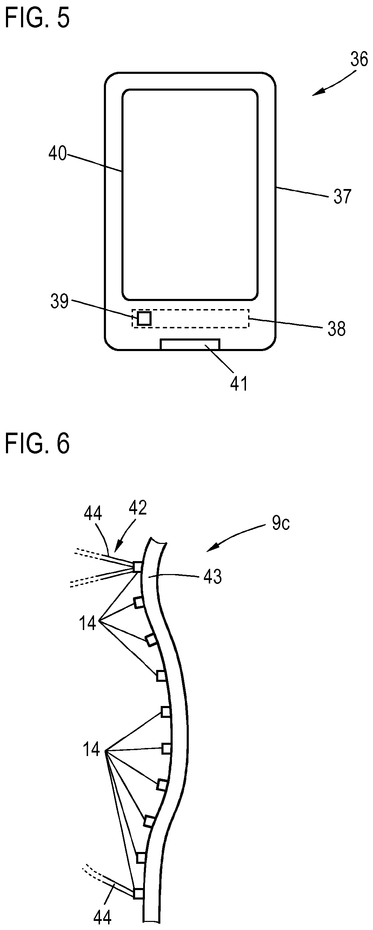

Diesbezüglich zeigt Fig. 5 eine mögliche Ausgestaltung, in der ein handgehaltenes Mobilgerät 36, hier ein Smartphone 37 oder Tablet, wenigstens teilweise als Auswerteeinrichtung 16, Ausgabeeinrichtung 20 und Einstelleinrichtung 21 verwendet wird. Das Mobilgerät 36 weist hierzu eine Recheneinrichtung 38, insbesondere umfassend wenigstens einen Prozessor, auf, auf der eine Applikation 39 (App), also ein Computerprogramm, vorgesehen ist, welches die entsprechenden Teile bzw. Komponenten eines medizinischen Hilfsmittels 9, 9a, 9b umsetzt.

Über die Applikation 39 kann beispielsweise ein Touchscreen 40 als Eingabemittel der Eingabeeinrichtung 21 eingesetzt werden, um gewünschte Bewegungsbereiche 6 bzw. zur automatischen Ermittlung derselben zu nutzende Vorgabedaten einzugeben. Ferner kann das Display des Touchscreens 40 genau wie ein Lautsprecher 41 auch als ein Ausgabemittel der Ausgabeeinrichtung 20 genutzt werden. Die Recheneinrichtung 38 kann wenigstens teilweise die Ermittlungseinheit 17, die Vergleichseinheit 18 und/oder die Unterstützungseinheit 19 realisieren.

Fig. 6 zeigt den Einsatz eines medizinischen Hilfsmittels 9c bei einer als Rückenorthese 42 ausgebildeten Behandlungseinrichtung 22. Die Rückenorthese 42, die der Wirbelsäule als Gelenk bzw. Gelenkgruppe zugeordnet ist, weist eine Rückenschiene 43 auf, die über ein Gurtsystem 44 gehaltert wird. Entlang der Rückenschiene 43 als Gelenkführungselement sind in diesem Fall eine größere Anzahl von Sensoren 14 angeordnet, um die Bewegung der Wirbelsäule möglichst genau zu vermessen. Bewegungsbereiche 4, 5 und 6 können beispielsweise durch lokale Bewegungsbegrenzungen oder dergleichen beschrieben werden.

Wiederum sind der Übersichtlichkeit halber weitere Komponenten des medizinischen Hilfsmittels 9c nicht gezeigt; die Rückenorthese 42 kann, muss aber nicht als Behandlungseinrichtung 22 dem medizinischen Hilfsmittel 9c zugehörig sein.

Es sei an dieser Stelle noch angemerkt, dass durch das Gurtsystem 44, die Bandage 34 sowie die Gurte 26 letztlich Umfassungsabschnitte gebildet sind, die bei Zugehörigkeit der Behandlungseinrichtung 22 zu dem entsprechenden medizinischen Hilfsmitteln 9, 9a, 9b, 9c zumindest bereichsweise als Anteile dienen können. Ferner sei darauf hingewiesen, dass ähnlich wie die Bandage 34 auch ein Strumpf oder ein anderes Kleidungsstück als Behandlungseinrichtung 22 bzw. Teil des Hilfsmittels 9, 9a, 9b, 9c herangezogen werden kann.

Zurückkehrend zu Fig. 2 kann die Auswerteeinrichtung 16 im Übrigen auch zusätzlich eine Klassifizierungseinheit 45 aufweisen, welche anhand der Erfassungsdaten der Erfassungseinrichtung 13 die aktuelle Bewegung in eine Bewegungsartklasse klassifizieren kann. Hierbei kann insbesondere vorgesehen sein, dass nur bei wenigstens einer bestimmten Bewegungsart Unterstützungshinweise ausgegeben werden, unterschiedliche Unterstützungshinweise bzw. unterschiedliche gewünschte Bewegungsbereiche 6 für unterschiedliche Bewegungsartklassen definiert werden und dergleichen. Beispielsweise kann es gewünscht sein, andere gewünschte Bewegungsbereiche 6 bzw. andere Unterstützungshinweise für ein Kniegelenk als Gelenk 1 beim Treppensteigen und beim normalen Laufen auf einer ebenen Fläche vorzusehen.

Die Erfassungsdaten, insbesondere über einen definierten zurückliegenden Zeitraum, können dabei hinreichend deutlich anzeigen, welche Bewegungsartklasse gerade genutzt wird.

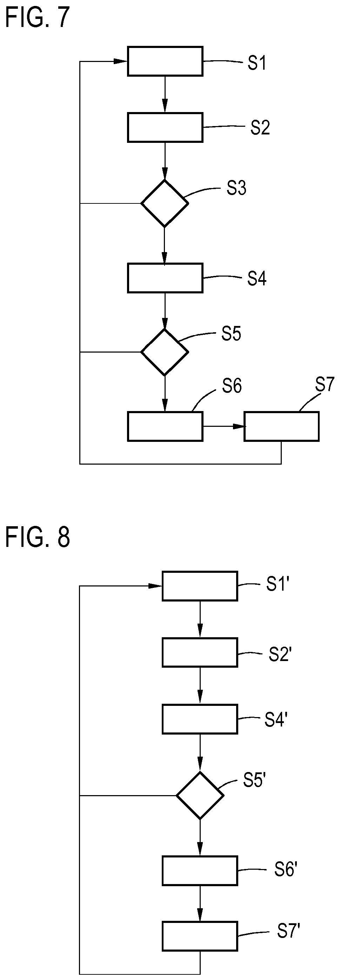

Mit Hinweis auf die Figuren 7 und 8 werden nun beispielhafte Ausführungsbeispiele eines Verfahrens, das durch die beschriebenen medizinischen Hilfsmittel 9, 9a, 9b, 9c durchgeführt werden kann, näher erläutert.

Im Ausführungsbeispiel gemäß Fig. 7 werden in einem Schritt S1 die Erfassungsdaten mittels der Erfassungseinrichtung 13 aufgenommen.

In einem Schritt S2 wird die Ermittlungseinheit 17 der Auswerteeinrichtung 16 verwendet, um den tatsächlich beim Tragen der Behandlungseinrichtung genutzten Bewegungsbereich 5 des Gelenks 1 aus den Erfassungsdaten zu ermitteln, wobei auch weitere Analysen der Erfassungsdaten durchgeführt werden. Im Fall des Ausführungsbeispiels der Fig. 7 wird nämlich nicht nur der aktuelle Bewegungszustand, sondern auch die unmittelbar zurückliegende Bewegungshistorie und/oder Dynamikparameter, beispielsweise bei einem Ein- oder Mehrachsgelenk eine Winkelgeschwindigkeit, berücksichtigt. Im vorliegenden Fall soll ein Knie bei einer zyklischen Bewegung genauer betrachtet werden. Diese besteht üblicherweise aus Abfolgen eines Extensionsvorgangs und eines Flexionsvorgangs, wobei diese beiden Vorgänge jeweils an Wendepunkten, die Begrenzungen des tatsächlich genutzten Bewegungsbereichs 5 darstellen, wechseln. Nähert sich der Bewegungszustand dem Wendepunkt, äußerst sich dies beispielsweise in einer Reduzierung der Winkelgeschwindigkeit. Dies ermöglicht es aber, bereits vor Erreichen des Wendepunktes festzustellen, dass man sich diesem Wendepunkt nähert, insbesondere auch welchem Wendepunkt, und zudem zu prädizieren, bis wohin der aktuelle Bewegungsvorgang voraussichtlich erfolgen wird, insbesondere also bis zu welchem Gelenkwinkel.

Basierend auf den im Schritt S2 ermittelten Informationen wird in einem Schritt S3 überprüft, ob eine relevante Bewegungsphase bzw. ein relevanter Bewegungszustand überhaupt vorliegen. Dies kann als ein erstes Hinweiskriterium aufgefasst werden, nachdem ja letztlich überprüft wird, ob überhaupt ein intuitiv erfassbarer, mithin sinnvoller Unterstützungshinweis im aktuellen Bewegungszustand bzw. der aktuellen Bewegungsphase möglich wäre. Ist dies nicht der Fall, wird wieder zu Schritt S1 zurückgekehrt, ansonsten wird mit Schritt S4 fortgefahren. Im Schritt S4 überprüft die Vergleichseinheit 18 der Auswerteeinrichtung 16, inwieweit im konkreten Beispiel der prädizierte Wendepunkt an die entsprechende Begrenzung 7 des gewünschten Bewegungsbereichs 6 herankommt. Im Beispiel des Knies als Einachsgelenk kann beispielsweise ein Winkelabstand zwischen der Begrenzung 7 und dem prädizierten Wendepunkt bestimmt werden. Selbstverständlich sind in anderen Ausführungsbeispielen auch komplexere Analysen/Nutzungsmaße denkbar.

In einem Schritt S5 kann im Rahmen eines weiteren Hinweiskriteriums überprüft werden, ob die im Schritt S4 ermittelte Abweichung von der Begrenzung 7 überhaupt einen Unterstützungshinweis erfordert. Ist dies nicht erforderlich, beispielsweise, weil die Begrenzung 7 ohnehin erreicht (oder sogar überschritten) wird, wird wieder zu Schritt S1 zurückgekehrt. Es sei darauf hingewiesen, dass es aber durchaus auch denkbar ist, einen bekräftigenden Unterstützungshinweis auch bei vollständiger bzw. fast vollständiger Nutzung des Bewegungsbereichs 6 auszugeben.

In einem Schritt S6 wird dann der Unterstützungshinweis in Abhängigkeit des Vergleichsergebnisses des Schrittes S4 ermittelt, um in einem Schritt S7 ausgegeben zu werden.

Dabei findet in Schritt S6 insbesondere eine Parametrierung des Unterstützungshinweises in Abhängigkeit der aus den Erfassungsdaten gesammelten Informationen und insbesondere des Vergleichsergebnisses statt. Dabei kann beispielsweise die Intensität eines intuitiv eine Weiterbewegung auf die Begrenzung 7 zu fördernden Unterstützungshinweises zunehmen, je weiter der Winkelabstand von der Begrenzung 7 ist. Wird beispielsweise eine Stimulationseinrichtung, wie das Vibrationskissen 29, und/oder ein Antriebsmittel 30 verwendet, kann eine stärkere Stimulation/ein stärkerer Unterstützungshinweis stattfinden, wenn der Person 10 eine längere Fortsetzung eines aktuellen Bewegungsvorgangs, beispielsweise Extension oder Flexion, empfohlen werden soll. Entsprechendes kann für akustische und/oder optische Unterstützungshinweise gelten; hierbei kann es jedoch auch zweckmäßig sein, intuitiv eine Annäherung an die Begrenzung 7 durch höhere Intensität zu vermitteln.

Fig. 8 zeigt ein zweites, alternativ oder auch in Kombination mit Fig. 7 einsetzbares Ausführungsbeispiel des Verfahrens.

Auch dort wird in einem Schritt S1 die Bewegung durch Aufnahme von Erfassungsdaten vermessen. In einem Schritt S2 erfolgt in der Ermittlungseinheit allerdings eine Betrachtung über vorliegend mehrere Bewegungszyklen, das bedeutet, es werden auch Erfassungsdaten der Vergangenheit, die die Bewegungshistorie in einem unmittelbar vor dem jetzigen Zeitpunkt liegenden Zeitraum beschreiben, herangezogen, so dass eine robustere Beschreibung des aktuell tatsächlich genutzten Bewegungsbereichs 5 resultieren kann. Dabei können beispielsweise, insbesondere auch gewichtete, Mittelwerte für Extrempositionen gebildet werden und dergleichen. Dabei wird eine kontinuierliche Aktualisierung mit allen neuen Erfassungsdaten vorgenommen, das bedeutet, es wird ein gleitender Zeitraum betrachtet, um möglichst schnell auf Veränderungen, insbesondere Verbesserungen oder Verschlechterungen hinsichtlich des gewünschten Bewegungsbereichs 6, reagieren zu können.

In einem Schritt S4' findet der Vergleich in der Vergleichseinheit 18 statt, nachdem in diesem Ausführungsbeispiel ohnehin eine kontinuierliche Information, als Leitung bzw. Führung der Person, durch Unterstützungshinweise gewünscht ist.

Nichtsdestotrotz kann in einem Schritt S5', der wiederum optional ist, eine Überprüfung von Hinweiskriterien dahingehend erfolgen, ob überhaupt ein Unterstützungshinweis erforderlich ist. Gerade bei einer letztlich gewünschten kontinuierlichen Führung der Person 10 zu einer verbesserten Ausnutzung des gewünschten Bewegungsbereichs 6 kann dann, wenn eine hinreichende Nutzung vorliegt, auch gewünscht sein, keine Unterstützungshinweise mehr auszugeben.

In einem Schritt S6' wird dann, analog zu dem Schritt S6 in Fig. 7, der Unterstützungshinweis ermittelt und im Schritt S7 ausgegeben. In diesem Ausführungsbeispiel ist es dabei bevorzugt, eine Tonfolge zu verwenden, deren Wiederholfrequenz abhängig vom Nutzungsgrad, hier insbesondere der Annäherung an die Begrenzungen 7, ist.

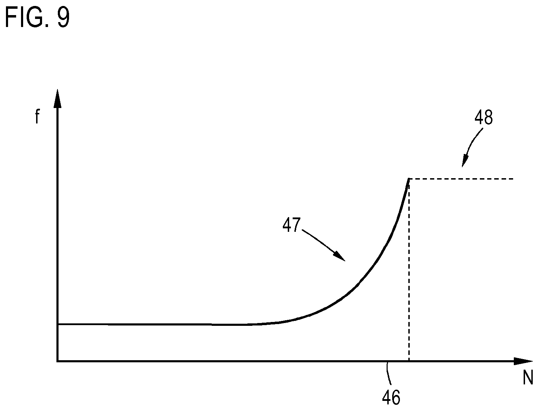

Eine derartige beispielhafte Abhängigkeit einer Wiederholfrequenz f eines Tones vom Nutzungsgrad N zeigt Fig. 9. Dabei deutet der Wert 46 eine im Wesentlichen vollständige Nutzung des gewünschten Bewegungsbereichs 6 an. Ersichtlich ist zunächst eine niedrige Wiederholfrequenz gegeben, die mit zunehmender Nutzung im Bereich 47 deutlich ansteigt, diese Annäherung an die Begrenzungen 7 mithin ähnlich wie ein "Parkwarner" intuitiv vermittelt. Ab dem Wert 46, Bereich 48, kann beispielsweise ein Dauerton ausgegeben werden und/oder der Unterstützungshinweis aufgrund des Erreichens der größtmöglichen Nutzung des gewünschten Bewegungsbereichs 6 deaktiviert werden.

Es sei abschließend noch angemerkt, dass es nicht nur in den Ausführungsbeispielen gemäß Fig. 7 und Fig. 8, sondern auch grundsätzlich bei allen Ausgestaltungen der Erfindung vorgesehen ist, die Unterstützungshinweise intuitiv der Bewegung zuordenbar während der Bewegung selbst auszugeben, insbesondere unmittelbar nach der Beurteilung. Dies verbessert die Zuordnung, insbesondere bei Reaktion auf bestimmte Bewegungsphasen bzw. Prädiktionen und somit auch den Leitungs- und Führungseffekt zur verbesserten Ausnutzung des gewünschten Bewegungsbereichs 6. Ferner ist noch allgemein darauf hinzuweisen, dass bevorzugt die Unterstützungshinweise die Bewegung selbst nicht stören bzw. unterbrechen, mithin intuitiv in die Bewegungsabläufe einfließen und den Benutzer so zu der verbesserten Ausnutzung des gewünschten Bewegungsbereichs 6 führen.

## Patentansprüche

1. Medizinisches Hilfsmittel (9, 9a, 9b, 9c) für ein Gelenk (1) einer Person (10), das wenigstens zwei an dem Gelenk (1) anzulegende, durch das Gelenk (1) gegeneinander bewegliche Anteile (11) und wenigstens eine Erfassungseinrichtung (13) zur Erfassung einer Relativbewegung der Anteile (11) aufweist, **gekennzeichnet durch** eine Auswerteeinrichtung (16), aufweisend:
- eine Ermittlungseinheit (17) zur Ermittlung eines tatsächlich beim Tragen des Hilfsmittels (9, 9a, 9b, 9c) genutzten Bewegungsbereichs (5) des Gelenks (1) aus Erfassungsdaten der Erfassungseinrichtung (13),
- eine Vergleichseinheit (18) zum Vergleich des genutzten Bewegungsbereichs (5) mit einem gewünschten Bewegungsbereich (6), und
- eine Unterstützungseinheit (19) zum Ermitteln eines die Person (10) zur möglichst weitgehenden Nutzung des gewünschten Bewegungsbereichs (6) führenden Unterstützungshinweises in Abhängigkeit des Vergleichsergebnisses, wobei das medizinische Hilfsmittel (9, 9a, 9b, 9c) ferner eine Ausgabeeinrichtung (20) zur Ausgabe des Unterstützungshinweises an die Person (10) aufweist, wobei die Auswerteeinrichtung (16) zur Ansteuerung der Ausgabeeinrichtung (20) zur Ausgabe des wenigstens einen Unterstützungshinweises während einer beurteilten Bewegung ausgebildet ist, **dadurch gekennzeichnet, dass** die Unterstützungseinheit (19) zur Auswahl
• wenigstens eines Ausgabeparameters wenigstens eines des wenigstens einen Unterstützungshinweises, wobei der Ausgabeparameter eine Intensität eines Tons und/oder eines Lichtsignals und/oder eine Lautstärke eines Tons ist,
• in Abhängigkeit eines aus dem Vergleichsergebnis ermittelten Nutzungsmaßes des gewünschten Bewegungsbereichs (6) und/oder Annäherungsmaßes an eine Grenze (7) des gewünschten Bewegungsbereichs (6) ausgebildet ist.

2. Medizinisches Hilfsmittel (9, 9a, 9b, 9c) nach Anspruch 1, **dadurch gekennzeichnet, dass** das Hilfsmittel (9, 9a, 9b, 9c) wenigstens einen strumpfartigen, über das Gelenk (1) zu ziehenden und/oder bandagenartig um das Gelenk (1) zu wickelnden und/oder als Kleidungsstück und/oder als Gurtsystem (44) ausgebildeten Umfassungsabschnitt und/oder wenigstens ein Gelenkführungselement (25), insbesondere wenigstens zwei gelenkig gekoppelte, starre Gelenkführungselemente (25), aufweist.

3. Medizinisches Hilfsmittel (9, 9a, 9b, 9c) nach Anspruch 1 oder 2, **dadurch gekennzeichnet, dass** die Erfassungseinrichtung (13) wenigstens einen in wenigstens einen Anteil (11) integrierten oder an wenigstens einem Anteil (11) und/oder einem mit einem Anteil (11) bewegten Körperbereich lösbar oder unlösbar befestigten oder befestigbaren Sensor (14) umfasst und/oder zur Messung eines Abstandes zwischen zwei Sensoren (14) und/oder Sensoranteilen und/oder eines Gelenkwinkels und/oder wenigstens einer Dynamikgröße der Gelenkbewegung, insbesondere einer Winkelgeschwindigkeit und/oder einer Winkelbeschleunigung, ausgebildet ist und/oder der wenigstens eine Sensor (14) ein Magnetismus und/oder Induktion nutzendes Messprinzip aufweist.

4. Medizinisches Hilfsmittel (9, 9a, 9b, 9c) nach einem der vorangehenden Ansprüche, **dadurch gekennzeichnet, dass** das Hilfsmittel (9, 9a, 9b, 9c) eine Einstelleinrichtung (21) zum Einstellen des gewünschten Bewegungsbereichs (6) des Gelenks (1), den die Person (10) beim Tragen des Hilfsmittels (9, 9a, 9b, 9c) nutzen soll, aufweist.

5. Medizinisches Hilfsmittel (9, 9a, 9b, 9c) nach Anspruch 4, **dadurch gekennzeichnet, dass** die Einstelleinrichtung (21) ein, insbesondere getrennt von einer durch die Anteile (11) gebildeten Behandlungseinrichtung (22) vorliegendes, elektronisches und/oder mechanisches Einstellmittel aufweist, insbesondere ein handhaltbares Mobilgerät (36) umfasst.

6. Medizinisches Hilfsmittel (9, 9a, 9b, 9c) nach Anspruch 5, **dadurch gekennzeichnet, dass** das Mobilgerät (36) zusätzlich eine als wenigstens ein Teil der Auswerteeinrichtung (16) ausgebildete Recheneinrichtung (38), auf der eine das Mobilgerät (36) als Einstellmittel und als der Teil der Auswerteeinrichtung (16) ausbildende Applikation (39) vorliegt, aufweist.

7. Medizinisches Hilfsmittel (9, 9a, 9b, 9c) nach einem der Ansprüche 4 bis 6, **dadurch gekennzeichnet, dass** die Einstelleinrichtung (21) zur zumindest teilweise automatischen Ermittlung des gewünschten Bewegungsbereichs (6) durch Auswertung von personenspezifischen Vorgabedaten und/oder die Gelenkbewegung des Gelenks (1) der Person (10) in einem zurückliegenden Zeitabschnitt beschreibende, mit der Erfassungseinrichtung (13) erfasste Historiendaten ausgebildet ist.

8. Medizinisches Hilfsmittel (9, 9a, 9b, 9c) nach einem der vorangehenden Ansprüche, **dadurch gekennzeichnet, dass** seitens der Unterstützungseinheit (19) zur Ermittlung des Unterstützungshinweises und/oder eines Ausgabezeitpunkts des Unterstützungshinweises eine Bewegungsphase, insbesondere eine Annäherung des Bewegungszustands an eine Extremposition, und/oder eine Bewegungshistorie des Gelenks (1) berücksichtigt wird.

9. Medizinisches Hilfsmittel (9, 9a, 9b, 9c) nach einem der vorangehenden Ansprüche, **dadurch gekennzeichnet, dass** die Unterstützungseinheit (19) zur Auswahl wenigstens eines weiteren Ausgabeparameters, insbesondere der Frequenz der Wiederholung des Tons und/oder des Lichtsignals, des wenigstens einen des wenigstens einen Unterstützungshinweises in Abhängigkeit des Nutzungsmaßes und/oder Annäherungsmaßes ausgebildet ist.

10. Medizinisches Hilfsmittel (9, 9a, 9b, 9c) nach einem der vorangehenden Ansprüche, **dadurch gekennzeichnet, dass** es ferner, insbesondere als Teil der Ausgabeeinrichtung (20), wenigstens eine durch die Auswerteeinrichtung (16) in Abhängigkeit des Vergleichsergebnisses ansteuerbare Stimulationseinrichtung für wenigstens einen dem Gelenk (1) zugeordneten Muskel der Person (10) und/oder wenigstens ein zur Veränderung der Relativposition der Anteile (11) ausgebildetes, durch die Auswerteeinrichtung (16) in Abhängigkeit des Vergleichsergebnisses ansteuerbares, elektrisches und/oder mechanisches Antriebsmittel (30) aufweist.

11. Medizinisches Hilfsmittel (9, 9a, 9b, 9c) nach einem der vorangehenden Ansprüche, **dadurch gekennzeichnet, dass** die Auswerteeinrichtung (16) eine Klassifizierungseinheit (45) aufweist, die anhand der Erfassungsdaten der Erfassungseinrichtung (13) die aktuelle Bewegung in eine Bewegungsartklasse klassifiziert, wobei der Unterstützungshinweis nur bei Vorliegen einer der zugeordneten Bewegungsart entsprechenden Bewegungsartklasse ausgegeben wird.

12. Medizinisches Hilfsmittel (9, 9a, 9b, 9c) nach einem der vorangehenden Ansprüche, **dadurch gekennzeichnet, dass** es eine Begrenzungseinrichtung (31) zur Begrenzung eines Bewegungsbereichs (2) des Gelenks (1) auf einen zulässigen Bewegungsbereich (4) aufweist.

## Claims

1. Medical aid (9, 9a, 9b, 9c) for a joint (1) of a person (10), the medical aid having at least two parts (11) which can be positioned on the joint (1), and are movable in relation to one another by the joint (1), and having at least one detection device (13) for detecting a relative movement of the parts (11), **characterized by** an evaluation device (16) having:
- a determination unit (17), by means of which a movement range (5) of the joint (1) that is actually in use when the aid (9, 9a, 9b, 9c) is being worn is determined from detection data from the detection device (13),
- a comparison unit (18), by means of which the movement range (5) used is compared with a desired movement range (6), and
- an assistance unit (19), by means of which an item of assistance information which causes the person (10) to make as extensive use as possible of the desired movement range (6) is determined in dependence on the comparison result, wherein the medical aid (9, 9a, 9b, 9c) also has an output device (20) for outputting the item of assistance information to the person (10), wherein the evaluation device (16) is designed to activate the output device (20) for outputting the at least one item of assistance information during an assessed movement, **characterized in that** the assistance unit (19) is designed for selecting
• at least one output parameter of at least one of the at least one item of assistance information, wherein the output parameter is an intensity of a sound and/or of a light signal and/or a volume of a sound,
• in dependence on an extent of use of the desired movement range (6), the extent of use being determined from the comparison result, and/or an extent of approach to a limit (7) of the desired movement range (6).

2. Medical aid (9, 9a, 9b, 9c) according to Claim 1, **characterized in that** the aid (9, 9a, 9b, 9c) has at least one stocking-like enclosure section to be pulled over the joint (1) and/or to be wrapped in a bandage-like manner around the joint (1) and/or in the form of an item of clothing and/or in the form of a strap system (44), and/or has at least one joint-guiding element (25), in particular at least two rigid joint-guiding elements (25) coupled in an articulated manner.

3. Medical aid (9, 9a, 9b, 9c) according to Claim 1 or 2, **characterized in that** the detection device (13) comprises at least one sensor (14), which is integrated in at least one part (11) or is fastened, or can be fastened, detachably or non-detachably on at least one part (11) and/or a body region moving with a part (11), and/or is designed for measuring a distance between two sensors (14) and/or sensor parts and/or for measuring a joint angle and/or at least one dynamic variable of the joint movement, in particular an angular velocity and/or an angular acceleration, and/or the at least one sensor (14) has a measuring principle which uses magnetism and/or induction.

4. Medical aid (9, 9a, 9b, 9c) according to one of the preceding claims, **characterized in that** the aid (9, 9a, 9b, 9c) has a setting device (21) for setting the desired movement range (6) of the joint (1) that the person (10) is to use when wearing the aid (9, 9a, 9b, 9c).

5. Medical aid (9, 9a, 9b, 9c) according to Claim 4, **characterized in that** the setting device (21) comprises an electronic and/or mechanical setting means, in particular a hand-held mobile device (36), which exists in particular independently of a treatment device (22) formed by the parts (11).

6. Medical aid (9, 9a, 9b, 9c) according to Claim 5, **characterized in that** the mobile device (36) additionally has a computing device (38), which is formed as at least part of the evaluation device (16) and on which there is an application (39), which makes the mobile device (36) a setting means and part of the evaluation device (16).

7. Medical aid (9, 9a, 9b, 9c) according to one of Claims 4 to 6, **characterized in that** the setting device (21) is designed for at least partly automatically determining the desired movement range (6) by evaluation of person-specific default data and/or historical data detected by the detection device (13) and describing the movement of the joint (1) of the person (10) in a previous period of time.

8. Medical aid (9, 9a, 9b, 9c) according to one of the preceding claims, **characterized in that** a movement phase, in particular an approach of the state of movement to an extreme position, and/or a movement history of the joint (1) is/are taken into account by the assistance unit (19) for determining the item of assistance information and/or an output time of the item of assistance information.

9. Medical aid (9, 9a, 9b, 9c) according to one of the preceding claims, **characterized in that** the assistance unit (19) is designed for selecting at least one further output parameter, in particular the frequency of repetition of the sound and/or of the light signal, of the at least one of the at least one item of assistance information in dependence on the extent of use and/or extent of approach.

10. Medical aid (9, 9a, 9b, 9c) according to one of the preceding claims, **characterized in that** it also has, in particular as part of the output device (20), at least one stimulation device, which can be activated by the evaluation device (16) in dependence on the comparison result and is intended for at least one muscle, associated with the joint (1), of the person (10), and/or at least one electrical and/or mechanical drive means (30), which is designed for changing the relative position of the parts (11) and can be activated by the evaluation device (16) in dependence on the comparison result.

11. Medical aid (9, 9a, 9b, 9c) according to one of the preceding claims, **characterized in that** the evaluation device (16) has a classification unit (45), which classifies the current movement into a class of movement types on the basis of the detection data from the detection device (13), wherein the item of assistance information is output only in the presence of a class of movement types which corresponds to the associated movement type.

12. Medical aid (9, 9a, 9b, 9c) according to one of the preceding claims, **characterized in that** it has a limiting device (31) for limiting a movement range (2) of the joint (1) to an admissible movement range (4).

## Revendications

1. Accessoire médical (9, 9a, 9b, 9c) pour une articulation (1) d'une personne (10), qui comporte au moins deux parties (11) à appliquer sur l'articulation (1) et pouvant être déplacées l'une par rapport à l'autre par l'articulation (1) et au moins un dispositif de détection (13) destiné à détecter un mouvement relatif des parties (11), **caractérisé par** un dispositif d'évaluation (16), comportant :
- une unité de détermination (17) destinée à déterminer une plage de mouvement (5) de l'articulation (1) effectivement utilisée lors du port de l'accessoire (9, 9a, 9b, 9c) à partir de données de détection du dispositif de détection (13),
- une unité de comparaison (18) destinée à comparer la plage de mouvement (5) utilisée à une plage de mouvement souhaitée (6), et
- une unité d'assistance (19) destinée à déterminer un conseil d'assistance conduisant la personne (10) à l'utilisation aussi large que possible de la plage de mouvement souhaitée (6) en fonction du résultat de la comparaison, l'accessoire médical (9, 9a, 9b, 9c) comportant en outre un dispositif de sortie (20) pour délivrer le conseil d'assistance à la personne (10), le dispositif d'évaluation (16) étant formé pour piloter le dispositif de sortie (20) pour délivrer l'au moins un conseil d'assistance pendant un mouvement jugé, **caractérisé en ce que** l'unité d'assistance (19) est formée pour choisir
• au moins un paramètre de sortie d'au moins un des conseils d'assistance, le paramètre de sortie étant une intensité d'un son et/ou d'un signal lumineux et/ou un volume d'un son,
• en fonction d'un degré d'utilisation de la plage de mouvement souhaitée (6) déterminée à partir du résultat de la comparaison et/ou d'un degré de rapprochement d'une limite (7) de la plage de mouvement souhaitée (6).

2. Accessoire médical (9, 9a, 9b, 9c) selon la revendication 1, **caractérisé en ce que** l'accessoire (9, 9a, 9b, 9c) comporte au moins une section d'enveloppe en forme de bas, à tirer sur l'articulation (1) et/ou à enrouler à la manière d'un bandage autour de l'articulation (1) et/ou formée comme un vêtement et/ou comme un système de ceinture (44) et/ou au moins un élément de guidage (25) d'articulation, en particulier au moins deux éléments de guidage (25) d'articulation rigides couplés de manière articulée.

3. Accessoire médical (9, 9a, 9b, 9c) selon la revendication 1 ou 2, **caractérisé en ce que** le dispositif de détection (13) comprend au moins un capteur (14) intégré dans au moins une partie (11) ou fixé ou pouvant être fixé de manière amovible ou inamovible sur au moins une partie (11) et/ou sur une zone corporelle déplacée avec une partie (11) et/ou est formé pour mesurer une distance entre deux capteurs (14) et/ou deux parties de capteur et/ou un angle d'articulation et/ou au moins une grandeur dynamique du mouvement de l'articulation, en particulier une vitesse angulaire et/ou une accélération angulaire, et/ou l'au moins un capteur (14) comporte un principe de mesure utilisant le magnétisme et/ou l'induction.

4. Accessoire médical (9, 9a, 9b, 9c) selon l'une des revendications précédentes, **caractérisé en ce que** l'accessoire (9, 9a, 9b, 9c) comporte un dispositif de réglage (21) destiné à régler la plage de mouvement souhaitée (6) de l'articulation (1) que la personne (10) doit utiliser lorsqu'elle porte l'accessoire (9, 9a, 9b, 9c).

5. Accessoire médical (9, 9a, 9b, 9c) selon la revendication 4, **caractérisé en ce que** le dispositif de réglage (21) comporte un moyen de réglage électronique et/ou mécanique, présent en particulier séparément d'un dispositif de traitement (22) formé par les parties (11), en particulier un appareil mobile (36) portatif.

6. Accessoire médical (9, 9a, 9b, 9c) selon la revendication 5, **caractérisé en ce que** l'appareil mobile (36) comporte en supplément un dispositif de calcul (38) formé comme au moins une partie du dispositif d'évaluation (16), sur lequel est présente une application (39) formant l'appareil mobile (36) comme un moyen de réglage et comme la partie du dispositif d'évaluation (16).

7. Accessoire médical (9, 9a, 9b, 9c) selon l'une des revendications 4 à 6, **caractérisé en ce que** le dispositif de réglage (21) est formé pour déterminer au moins en partie automatiquement la plage de mouvement souhaitée (6) en évaluant des données de spécification spécifiques à la personne et/ou des données d'historique décrivant le mouvement de l'articulation (1) de la personne (10) dans un laps de temps antérieur, détectées avec le dispositif de détection (13).

8. Accessoire médical (9, 9a, 9b, 9c) selon l'une des revendications précédentes, **caractérisé en ce que** l'unité d'assistance (19) tient compte d'une phase de mouvement, en particulier d'un rapprochement de l'état de mouvement vers une position extrême et/ou d'un historique de mouvement de l'articulation (1) pour déterminer le conseil d'assistance et/ou un moment de sortie du conseil d'assistance.

9. Accessoire médical (9, 9a, 9b, 9c) selon l'une des revendications précédentes, **caractérisé en ce que** l'unité d'assistance (19) est formée pour sélectionner au moins un autre paramètre de sortie, en particulier la fréquence de répétition du son et/ou du signal lumineux, de l'au moins un conseil d'assistance en fonction du degré d'utilisation et/ou du degré de rapprochement.

10. Accessoire médical (9, 9a, 9b, 9c) selon l'une des revendications précédentes, **caractérisé en ce qu'**il comporte en outre, en particulier en tant que partie du dispositif de sortie (20), au moins un dispositif de stimulation pouvant être piloté par le dispositif d'évaluation (16) en fonction du résultat de la comparaison, pour au moins un muscle de la personne (10) associé à l'articulation (1) et/ou au moins un moyen d'entraînement (30) électrique et/ou mécanique formé pour modifier la position relative des parties (11), pouvant être piloté par le dispositif d'évaluation (16) en fonction du résultat de la comparaison.

11. Accessoire médical (9, 9a, 9b, 9c) selon l'une des revendications précédentes, **caractérisé en ce que** le dispositif d'évaluation (16) comporte une unité de classification (45), qui classe le mouvement actuel dans une classe de type de mouvement à l'aide des données de détection du dispositif de détection (13), le conseil d'assistance n'étant délivré que lorsqu'une classe de type de mouvement correspondant au type de mouvement associé est présente.

12. Accessoire médical (9, 9a, 9b, 9c) selon l'une des revendications précédentes, **caractérisé en ce qu'**il comporte un dispositif de limitation (31) destiné à limiter une plage de mouvement (2) de l'articulation (1) à une plage de mouvement admissible (4).
